(19) Europäisches Patentamt / European Patent Office / Office européen des brevets

(11) **EP 4 786 484 A1**

(12) **EUROPEAN PATENT APPLICATION**
published in accordance with Art. 153(4) EPC

(43) Date of publication:
**05.08.2026 Bulletin 2026/32**

(21) Application number: **24870750.7**

(22) Date of filing: **25.09.2024**

(51) International Patent Classification (IPC):
***C07K 5/12*** (2006.01) ***C07K 7/64*** (2006.01)
***A61K 8/64*** (2006.01) ***A61Q 19/00*** (2006.01)
***A61Q 5/00*** (2006.01)

(52) Cooperative Patent Classification (CPC):
**A61K 8/64; A61Q 5/00; A61Q 19/00; C07K 5/12; C07K 7/64**

(86) International application number:
**PCT/CN2024/120959**

(87) International publication number:
**WO 2025/067206 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **25.09.2023 CN 202311247312**
**19.09.2024 CN 202411310856**

(71) Applicant: **Shenzhen Zhongge Biological Technology Co., Ltd.**
**Futian District**
**Shenzhen, Guangdong 518017 (CN)**

(72) Inventors:
- **LI, Hongmei**
  **Shenzhen, Guangdong 518017 (CN)**
- **SUN, Jixue**
  **Shenzhen, Guangdong 518017 (CN)**
- **LIN, Sheng**
  **Shenzhen, Guangdong 518017 (CN)**
- **GUI, Linna**
  **Shenzhen, Guangdong 518017 (CN)**
- **MA, Songling**
  **Shenzhen, Guangdong 518017 (CN)**
- **XIE, Dewei**
  **Shenzhen, Guangdong 518017 (CN)**
- **MA, Jin**
  **Shenzhen, Guangdong 518017 (CN)**
- **ZHAO, Liyu**
  **Shenzhen, Guangdong 518017 (CN)**
- **LUO, Jie**
  **Shenzhen, Guangdong 518017 (CN)**
- **LI, Wenting**
  **Shenzhen, Guangdong 518017 (CN)**
- **CHEN, Bin**
  **Shenzhen, Guangdong 518017 (CN)**
- **WANG, Shaohui**
  **Shenzhen, Guangdong 518017 (CN)**
- **ZHANG, Peiyu**
  **Shenzhen, Guangdong 518017 (CN)**
- **WEN, Shuhao**
  **Shenzhen, Guangdong 518017 (CN)**

(74) Representative: **Botti & Ferrari S.p.A.**
**Via Cappellini, 11**
**20124 Milano (IT)**

Remarks:
The complete document including Reference Table(s) and the Sequence Listing(s) can be downloaded from the EPO website

# (54) CYCLIC PEPTIDE COMPOUND AND USE THEREOF

(57) The present invention relates to a cyclic peptide compound and use thereof, specifically provides a cyclic peptide compound having a novel structure represented by formula I and an efficacy comparable to or even better than that of GHK tripeptide, and use of said cyclic peptide compound in a dermatological or cosmetic composition for stimulating hair growth or anti-aging of the skin.

*AA1-AA2-GLY-HIS-LYS-AA4-AA3*    Formula I



Processed by Luminess, 75001 PARIS (FR)

## Description

### REFERENCE TO RELATED APPLICATION

The present application claims priority to Chinese Patent Application CN 202311247312.8 filed on September 25, 2023 and Chinese Patent Application CN 202411310856.9 filed on September 19, 2024, the contents of which are incorporated herein by reference in their entirety and for all purposes.

### TECHNICAL FIELD

The present invention relates to the field of biomedicine or the field of cosmetic products, specifically to cyclic peptide compounds and uses thereof.

### BACKGROUND ART

GHK is a tripeptide molecule, also known as tripeptide or tripeptide-1, with a sequence of glycyl-histidyl-lysine, which is a hydrolysate of collagen. It is naturally found in the blood, saliva and urine of a person. GHK is known for its ability to promote production of type I collagen fragments. Its mechanism of action can be simply understood as when a collagen protein is naturally decomposed in skin, the resulting peptide fragments signal to the skin that it should start working and produce some pretty new collagen proteins.

GHK has a high affinity for a divalent copper ion, and can spontaneously bind to the divalent copper ion to form a complex GHK-Cu, the latter is also known as the blue-copper peptide because of the blue colour of the aqueous solution and is the main form of GHK that exerts efficacy in vivo. Its functions mainly include: (1) the chemotaxis of repairing cells, such as macrophages, mast cells, capillary cells; (2) anti-inflammatory effects (inhibiting free radicals, thromboxane formation, transforming growth factor beta-1, tumor necrosis factor alpha, and protein glycation, simultaneously increasing superoxide dismutase, dilating blood vessels, blocking UV damage to cutaneous keratinocytes, and improving fibroblast recovery after X-ray therapy, etc.); (3) increasing the synthesis of collagen proteins, elastin, hyaluronic acid, metalloprotease, anti-protease, vascular endothelial growth factor, fibroblast growth factor 2, nerve growth factor, neutrophil 3 and neutrophil 4, and erythropoietin; (4) increasing the proliferation of fibroblasts and keratinocytes; nerve growth, angiogenesis, and hair follicle size. Control studies of aged skin showed that GHK-Cu can tighten the skin, improve elasticity and compactness, and reduce fine lines, wrinkles, photo-damages and hyperpigmentation, and can also promote hair growth and improve the success rate of hair transplantation.

### SUMMARY OF THE INVENTION

The present invention provides a cyclic peptide compound having a novel structure and an efficacy comparable to or even better than that of GHK tripeptide, and use of said cyclic peptide compound in a dermatological or cosmetic composition for stimulating hair growth or anti-aging of the skin.

For this reason, in a first aspect of the invention, the present invention provides a cyclic peptide compound represented by formula I, or a stereoisomer thereof,

*AA1-AA2-GLY-HIS-LYS-AA4-AA3*     Formula I

wherein:

AA1 is an amino acid residue;
AA2 is absent or is an amino acid residue;
AA3 is an amino acid residue;
AA4 is absent or is an amino acid residue;
* indicates that the N-terminus and the C-terminus of formula I are linked to form a cyclic peptide;
the amino acid is a natural amino acid or a non-natural amino acid;
the amino acid is of D-configuration or L-configuration.

It should be noted that the formula I has the N-terminus on the left side and the C-terminus on the right side.

In some embodiments, the amino acid is of L-configuration.

In some embodiments, the amino acid is a natural amino acid.

In some embodiments, the amino acid is a hydrophobic natural amino acid.

In some embodiments, the amino acid is selected from the group consisting of alanine (ALA; A), glycine (GLY; G),

isoleucine (ILE; I), leucine (LEU; L), methionine (MET; M), phenylalanine (PHE; F), proline (PRO; P), tryptophan (TRP; W), and valine (VAL; V).

In some embodiments, AA1 is selected from the group consisting of a phenylalanine residue, an alanine residue, a methionine residue, a leucine residue, a tryptophan residue, an isoleucine residue, a proline residue, a glycine residue, and a valine residue.

In some embodiments, AA1 is selected from the group consisting of an alanine residue, a methionine residue, a phenylalanine residue, a leucine residue, a tryptophan residue, an isoleucine residue, a proline residue, and a glycine residue.

In some embodiments, AA1 is selected from the group consisting of a methionine residue, an alanine residue, a phenylalanine residue, a glycine residue, and a proline residue.

In some embodiments, AA2 is an amino acid residue.

In some embodiments, AA2 is selected from the group consisting of a phenylalanine residue, an alanine residue, a methionine residue, a leucine residue, a tryptophan residue, an isoleucine residue, a proline residue, a glycine residue, and a valine residue.

In some embodiments, AA2 is selected from the group consisting of a valine residue, a proline residue, a leucine residue, an alanine residue, an isoleucine residue, a methionine residue, and a tryptophan residue.

In some embodiments, AA2 is selected from the group consisting of a proline residue, a leucine residue, a valine residue, an isoleucine residue, and a tryptophan residue.

In some embodiments, AA3 is selected from the group consisting of a phenylalanine residue, an alanine residue, a methionine residue, a leucine residue, a tryptophan residue, an isoleucine residue, a proline residue, a glycine residue, and a valine residue.

In some embodiments, AA3 is selected from the group consisting of a phenylalanine residue, an alanine residue, a methionine residue, a leucine residue, a tryptophan residue, an isoleucine residue, a glycine residue, and a valine residue.

In some embodiments, AA3 is selected from the group consisting of a phenylalanine residue, an alanine residue, a methionine residue, a leucine residue, an isoleucine residue, and a valine residue.

In some embodiments, AA3 is selected from the group consisting of an isoleucine residue, a leucine residue, a phenylalanine residue, a methionine residue, and a valine residue.

In some embodiments, AA4 is absent.

In some embodiments, the cyclic peptide compound has a structure represented by formula I-1,

*AA3-AA1-AA2-GLY-HIS-LYS* Formula I-1

wherein:

AA1 is selected from the group consisting of a phenylalanine residue, an alanine residue, a methionine residue, a leucine residue, a tryptophan residue, an isoleucine residue, a proline residue, a glycine residue, and a valine residue; and/or

AA2 is selected from the group consisting of a phenylalanine residue, an alanine residue, a methionine residue, a leucine residue, a tryptophan residue, an isoleucine residue, a proline residue, a glycine residue, and a valine residue; and/or

AA3 is selected from the group consisting of a phenylalanine residue, an alanine residue, a methionine residue, a leucine residue, a tryptophan residue, an isoleucine residue, a proline residue, a glycine residue, and a valine residue;

* indicates that the N-terminus and the C-terminus of the formula I-1 are linked to form a cyclic peptide;

the amino acid is of D-configuration or L-configuration.

It should be noted that the formula I-1 has the N-terminus on the left side and the C-terminus on the right side.

In some embodiments, the amino acid is of L-configuration.

In some embodiments, AA1 is selected from the group consisting of a phenylalanine residue, an alanine residue, a methionine residue, a leucine residue, a tryptophan residue, an isoleucine residue, a proline residue, and a glycine residue.

In some embodiments, AA1 is selected from the group consisting of a methionine residue, an alanine residue, a phenylalanine residue, a glycine residue, and a proline residue.

In some embodiments, AA2 is selected from the group consisting of an alanine residue, a methionine residue, a leucine residue, a tryptophan residue, an isoleucine residue, a proline residue, and a valine residue.

In some embodiments, AA2 is selected from the group consisting of a proline residue, a leucine residue, a valine residue, an isoleucine residue, and a tryptophan residue.

In some embodiments, AA3 is selected from the group consisting of a phenylalanine residue, an alanine residue,

a methionine residue, a leucine residue, a tryptophan residue, an isoleucine residue, a glycine residue, and a valine residue.

In some embodiments, AA3 is selected from the group consisting of a phenylalanine residue, an alanine residue, a methionine residue, a leucine residue, an isoleucine residue, and a valine residue.

In some embodiments, AA3 is selected from the group consisting of an isoleucine residue, a leucine residue, a phenylalanine residue, a methionine residue, and a valine residue.

In some embodiments, the structural formula of the cyclic peptide compound is as shown in Table A.

Table A: The structural formula of the cyclic peptide compound (all amino acids are of L-configuration)

| SEQ ID NO: | The structural formula of the cyclic peptide compound |
|---|---|
| 1 | ALA ALA ALA GLY HIS LYS |
| 2 | ALA ALA GLY GLY HIS LYS |
| 3 | ALA ALA ILE GLY HIS LYS |
| 4 | ALA ALA LEU GLY HIS LYS |
| 5 | ALA ALA MET GLY HIS LYS |
| 6 | ALA ALA PHE GLY HIS LYS |
| 7 | ALA ALA PRO GLY HIS LYS |
| 8 | ALA ALA TRP GLY HIS LYS |
| 9 | ALA ALA VAL GLY HIS LYS |
| 10 | ALA GLY ALA GLY HIS LYS |
| 11 | ALA GLY GLY GLY HIS LYS |
| 12 | ALA GLY ILE GLY HIS LYS |
| 13 | ALA GLY LEU GLY HIS LYS |
| 14 | ALA GLY MET GLY HIS LYS |
| 15 | ALA GLY PHE GLY HIS LYS |
| 16 | ALA GLY PRO GLY HIS LYS |
| 17 | ALA GLY TRP GLY HIS LYS |
| 18 | ALA GLY VAL GLY HIS LYS |
| 19 | ALA ILE ALA GLY HIS LYS |
| 20 | ALA ILE GLY GLY HIS LYS |
| 21 | ALA ILE ILE GLY HIS LYS |
| 22 | ALA ILE LEU GLY HIS LYS |
| 23 | ALA ILE MET GLY HIS LYS |
| 24 | ALA ILE PHE GLY HIS LYS |
| 25 | ALA ILE PRO GLY HIS LYS |
| 26 | ALA ILE TRP GLY HIS LYS |
| 27 | ALA ILE VAL GLY HIS LYS |
| 28 | ALA LEU ALA GLY HIS LYS |
| 29 | ALA LEU GLY GLY HIS LYS |
| 30 | ALA LEU ILE GLY HIS LYS |
| 31 | ALA LEU LEU GLY HIS LYS |
| 32 | ALA LEU MET GLY HIS LYS |
| 33 | ALA LEU PHE GLY HIS LYS |
| 34 | ALA LEU PRO GLY HIS LYS |

(continued)

| SEQ ID NO: | The structural formula of the cyclic peptide compound |
|---|---|
| 35 | ALA LEU TRP GLY HIS LYS |
| 36 | ALA LEU VAL GLY HIS LYS |
| 37 | ALA MET ALA GLY HIS LYS |
| 38 | ALA MET GLY GLY HIS LYS |
| 39 | ALA MET ILE GLY HIS LYS |
| 40 | ALA MET LEU GLY HIS LYS |
| 41 | ALA MET MET GLY HIS LYS |
| 42 | ALA MET PHE GLY HIS LYS |
| 43 | ALA MET PRO GLY HIS LYS |
| 44 | ALA MET TRP GLY HIS LYS |
| 45 | ALA MET VAL GLY HIS LYS |
| 46 | ALA PHE ALA GLY HIS LYS |
| 47 | ALA PHE GLY GLY HIS LYS |
| 48 | ALA PHE ILE GLY HIS LYS |
| 49 | ALA PHE LEU GLY HIS LYS |
| 50 | ALA PHE MET GLY HIS LYS |
| 51 | ALA PHE PHE GLY HIS LYS |
| 52 | ALA PHE PRO GLY HIS LYS |
| 53 | ALA PHE TRP GLY HIS LYS |
| 54 | ALA PHE VAL GLY HIS LYS |
| 55 | ALA PRO ALA GLY HIS LYS |
| 56 | ALA PRO GLY GLY HIS LYS |
| 57 | ALA PRO ILE GLY HIS LYS |
| 58 | ALA PRO LEU GLY HIS LYS |
| 59 | ALA PRO MET GLY HIS LYS |
| 60 | ALA PRO PHE GLY HIS LYS |
| 61 | ALA PRO PRO GLY HIS LYS |
| 62 | ALA PRO TRP GLY HIS LYS |
| 63 | ALA PRO VAL GLY HIS LYS |
| 64 | ALA TRP ALA GLY HIS LYS |
| 65 | ALA TRP GLY GLY HIS LYS |
| 66 | ALA TRP ILE GLY HIS LYS |
| 67 | ALA TRP LEU GLY HIS LYS |
| 68 | ALA TRP MET GLY HIS LYS |
| 69 | ALA TRP PHE GLY HIS LYS |
| 70 | ALA TRP PRO GLY HIS LYS |
| 71 | ALA TRP TRP GLY HIS LYS |
| 72 | ALA TRP VAL GLY HIS LYS |
| 73 | ALA VAL ALA GLY HIS LYS |

(continued)

| SEQ ID NO: | The structural formula of the cyclic peptide compound |
|---|---|
| 74 | ALA VAL GLY GLY HIS LYS |
| 75 | ALA VAL ILE GLY HIS LYS |
| 76 | ALA VAL LEU GLY HIS LYS |
| 77 | ALA VAL MET GLY HIS LYS |
| 78 | ALA VAL PHE GLY HIS LYS |
| 79 | ALA VAL PRO GLY HIS LYS |
| 80 | ALA VAL TRP GLY HIS LYS |
| 81 | ALA VAL VAL GLY HIS LYS |
| 82 | GLY ALA ALA GLY HIS LYS |
| 83 | GLY ALA GLY GLY HIS LYS |
| 84 | GLY ALA ILE GLY HIS LYS |
| 85 | GLY ALA LEU GLY HIS LYS |
| 86 | GLY ALA MET GLY HIS LYS |
| 87 | GLY ALA PHE GLY HIS LYS |
| 88 | GLY ALA PRO GLY HIS LYS |
| 89 | GLY ALA TRP GLY HIS LYS |
| 90 | GLY ALA VAL GLY HIS LYS |
| 91 | GLY GLY ALA GLY HIS LYS |
| 92 | GLY GLY GLY GLY HIS LYS |
| 93 | GLY GLY ILE GLY HIS LYS |
| 94 | GLY GLY LEU GLY HIS LYS |
| 95 | GLY GLY MET GLY HIS LYS |
| 96 | GLY GLY PHE GLY HIS LYS |
| 97 | GLY GLY PRO GLY HIS LYS |
| 98 | GLY GLY TRP GLY HIS LYS |
| 99 | GLY GLY VAL GLY HIS LYS |
| 100 | GLY ILE ALA GLY HIS LYS |
| 101 | GLY ILE GLY GLY HIS LYS |
| 102 | GLY ILE ILE GLY HIS LYS |
| 103 | GLY ILE LEU GLY HIS LYS |
| 104 | GLY ILE MET GLY HIS LYS |
| 105 | GLY ILE PHE GLY HIS LYS |
| 106 | GLY ILE PRO GLY HIS LYS |
| 107 | GLY ILE TRP GLY HIS LYS |
| 108 | GLY ILE VAL GLY HIS LYS |
| 109 | GLY LEU ALA GLY HIS LYS |
| 110 | GLY LEU GLY GLY HIS LYS |
| 111 | GLY LEU ILE GLY HIS LYS |
| 112 | GLY LEU LEU GLY HIS LYS |

(continued)

| SEQ ID NO: | The structural formula of the cyclic peptide compound |
|---|---|
| 113 | GLY LEU MET GLY HIS LYS |
| 114 | GLY LEU PHE GLY HIS LYS |
| 115 | GLY LEU PRO GLY HIS LYS |
| 116 | GLY LEU TRP GLY HIS LYS |
| 117 | GLY LEU VAL GLY HIS LYS |
| 118 | GLY MET ALA GLY HIS LYS |
| 119 | GLY MET GLY GLY HIS LYS |
| 120 | GLY MET ILE GLY HIS LYS |
| 121 | GLY MET LEU GLY HIS LYS |
| 122 | GLY MET MET GLY HIS LYS |
| 123 | GLY MET PHE GLY HIS LYS |
| 124 | GLY MET PRO GLY HIS LYS |
| 125 | GLY MET TRP GLY HIS LYS |
| 126 | GLY MET VAL GLY HIS LYS |
| 127 | GLY PHE ALA GLY HIS LYS |
| 128 | GLY PHE GLY GLY HIS LYS |
| 129 | GLY PHE ILE GLY HIS LYS |
| 130 | GLY PHE LEU GLY HIS LYS |
| 131 | GLY PHE MET GLY HIS LYS |
| 132 | GLY PHE PHE GLY HIS LYS |
| 133 | GLY PHE PRO GLY HIS LYS |
| 134 | GLY PHE TRP GLY HIS LYS |
| 135 | GLY PHE VAL GLY HIS LYS |
| 136 | GLY PRO ALA GLY HIS LYS |
| 137 | GLY PRO GLY GLY HIS LYS |
| 138 | GLY PRO ILE GLY HIS LYS |
| 139 | GLY PRO LEU GLY HIS LYS |
| 140 | GLY PRO MET GLY HIS LYS |
| 141 | GLY PRO PHE GLY HIS LYS |
| 142 | GLY PRO PRO GLY HIS LYS |
| 143 | GLY PRO TRP GLY HIS LYS |
| 144 | GLY PRO VAL GLY HIS LYS |
| 145 | GLY TRP ALA GLY HIS LYS |
| 146 | GLY TRP GLY GLY HIS LYS |
| 147 | GLY TRP ILE GLY HIS LYS |
| 148 | GLY TRP LEU GLY HIS LYS |
| 149 | GLY TRP MET GLY HIS LYS |
| 150 | GLY TRP PHE GLY HIS LYS |
| 151 | GLY TRP PRO GLY HIS LYS |

(continued)

| SEQ ID NO: | The structural formula of the cyclic peptide compound |
| --- | --- |
| 152 | GLY TRP TRP GLY HIS LYS |
| 153 | GLY TRP VAL GLY HIS LYS |
| 154 | GLY VAL ALA GLY HIS LYS |
| 155 | GLY VAL GLY GLY HIS LYS |
| 156 | GLY VAL ILE GLY HIS LYS |
| 157 | GLY VAL LEU GLY HIS LYS |
| 158 | GLY VAL MET GLY HIS LYS |
| 159 | GLY VAL PHE GLY HIS LYS |
| 160 | GLY VAL PRO GLY HIS LYS |
| 161 | GLY VAL TRP GLY HIS LYS |
| 162 | GLY VAL VAL GLY HIS LYS |
| 163 | ILE ALA ALA GLY HIS LYS |
| 164 | ILE ALA GLY GLY HIS LYS |
| 165 | ILE ALA ILE GLY HIS LYS |
| 166 | ILE ALA LEU GLY HIS LYS |
| 167 | ILE ALA MET GLY HIS LYS |
| 168 | ILE ALA PHE GLY HIS LYS |
| 169 | ILE ALA PRO GLY HIS LYS |
| 170 | ILE ALA TRP GLY HIS LYS |
| 171 | ILE ALA VAL GLY HIS LYS |
| 172 | ILE GLY ALA GLY HIS LYS |
| 173 | ILE GLY GLY GLY HIS LYS |
| 174 | ILE GLY ILE GLY HIS LYS |
| 175 | ILE GLY LEU GLY HIS LYS |
| 176 | ILE GLY MET GLY HIS LYS |
| 177 | ILE GLY PHE GLY HIS LYS |
| 178 | ILE GLY PRO GLY HIS LYS |
| 179 | ILE GLY TRP GLY HIS LYS |
| 180 | ILE GLY VAL GLY HIS LYS |
| 181 | ILE ILE ALA GLY HIS LYS |
| 182 | ILE ILE GLY GLY HIS LYS |
| 183 | ILE ILE ILE GLY HIS LYS |
| 184 | ILE ILE LEU GLY HIS LYS |
| 185 | ILE ILE MET GLY HIS LYS |
| 186 | ILE ILE PHE GLY HIS LYS |
| 187 | ILE ILE PRO GLY HIS LYS |
| 188 | ILE ILE TRP GLY HIS LYS |
| 189 | ILE ILE VAL GLY HIS LYS |
| 190 | ILE LEU ALA GLY HIS LYS |

(continued)

| SEQ ID NO: | The structural formula of the cyclic peptide compound |
|---|---|
| 191 | ILE LEU GLY GLY HIS LYS |
| 192 | ILE LEU ILE GLY HIS LYS |
| 193 | ILE LEU LEU GLY HIS LYS |
| 194 | ILE LEU MET GLY HIS LYS |
| 195 | ILE LEU PHE GLY HIS LYS |
| 196 | ILE LEU PRO GLY HIS LYS |
| 197 | ILE LEU TRP GLY HIS LYS |
| 198 | ILE LEU VAL GLY HIS LYS |
| 199 | ILE MET ALA GLY HIS LYS |
| 200 | ILE MET GLY GLY HIS LYS |
| 201 | ILE MET ILE GLY HIS LYS |
| 202 | ILE MET LEU GLY HIS LYS |
| 203 | ILE MET MET GLY HIS LYS |
| 204 | ILE MET PHE GLY HIS LYS |
| 205 | ILE MET PRO GLY HIS LYS |
| 206 | ILE MET TRP GLY HIS LYS |
| 207 | ILE MET VAL GLY HIS LYS |
| 208 | ILE PHE ALA GLY HIS LYS |
| 209 | ILE PHE GLY GLY HIS LYS |
| 210 | ILE PHE ILE GLY HIS LYS |
| 211 | ILE PHE LEU GLY HIS LYS |
| 212 | ILE PHE MET GLY HIS LYS |
| 213 | ILE PHE PHE GLY HIS LYS |
| 214 | ILE PHE PRO GLY HIS LYS |
| 215 | ILE PHE TRP GLY HIS LYS |
| 216 | ILE PHE VAL GLY HIS LYS |
| 217 | ILE PRO ALA GLY HIS LYS |
| 218 | ILE PRO GLY GLY HIS LYS |
| 219 | ILE PRO ILE GLY HIS LYS |
| 220 | ILE PRO LEU GLY HIS LYS |
| 221 | ILE PRO MET GLY HIS LYS |
| 222 | ILE PRO PHE GLY HIS LYS |
| 223 | ILE PRO PRO GLY HIS LYS |
| 224 | ILE PRO TRP GLY HIS LYS |
| 225 | ILE PRO VAL GLY HIS LYS |
| 226 | ILE TRP ALA GLY HIS LYS |
| 227 | ILE TRP GLY GLY HIS LYS |
| 228 | ILE TRP ILE GLY HIS LYS |
| 229 | ILE TRP LEU GLY HIS LYS |

(continued)

| SEQ ID NO: | The structural formula of the cyclic peptide compound |
|---|---|
| 230 | ILE TRP MET GLY HIS LYS |
| 231 | ILE TRP PHE GLY HIS LYS |
| 232 | ILE TRP PRO GLY HIS LYS |
| 233 | ILE TRP TRP GLY HIS LYS |
| 234 | ILE TRP VAL GLY HIS LYS |
| 235 | ILE VAL ALA GLY HIS LYS |
| 236 | ILE VAL GLY GLY HIS LYS |
| 237 | ILE VAL ILE GLY HIS LYS |
| 238 | ILE VAL LEU GLY HIS LYS |
| 239 | ILE VAL MET GLY HIS LYS |
| 240 | ILE VAL PHE GLY HIS LYS |
| 241 | ILE VAL PRO GLY HIS LYS |
| 242 | ILE VAL TRP GLY HIS LYS |
| 243 | ILE VAL VAL GLY HIS LYS |
| 244 | LEU ALA ALA GLY HIS LYS |
| 245 | LEU ALA GLY GLY HIS LYS |
| 246 | LEU ALA ILE GLY HIS LYS |
| 247 | LEU ALA LEU GLY HIS LYS |
| 248 | LEU ALA MET GLY HIS LYS |
| 249 | LEU ALA PHE GLY HIS LYS |
| 250 | LEU ALA PRO GLY HIS LYS |
| 251 | LEU ALA TRP GLY HIS LYS |
| 252 | LEU ALA VAL GLY HIS LYS |
| 253 | LEU GLY ALA GLY HIS LYS |
| 254 | LEU GLY GLY GLY HIS LYS |
| 255 | LEU GLY ILE GLY HIS LYS |
| 256 | LEU GLY LEU GLY HIS LYS |
| 257 | LEU GLY MET GLY HIS LYS |
| 258 | LEU GLY PHE GLY HIS LYS |
| 259 | LEU GLY PRO GLY HIS LYS |
| 260 | LEU GLY TRP GLY HIS LYS |
| 261 | LEU GLY VAL GLY HIS LYS |
| 262 | LEU ILE ALA GLY HIS LYS |
| 263 | LEU ILE GLY GLY HIS LYS |
| 264 | LEU ILE ILE GLY HIS LYS |
| 265 | LEU ILE LEU GLY HIS LYS |
| 266 | LEU ILE MET GLY HIS LYS |
| 267 | LEU ILE PHE GLY HIS LYS |
| 268 | LEU ILE PRO GLY HIS LYS |

(continued)

| SEQ ID NO: | The structural formula of the cyclic peptide compound |
|---|---|
| 269 | LEU ILE TRP GLY HIS LYS |
| 270 | LEU ILE VAL GLY HIS LYS |
| 271 | LEU LEU ALA GLY HIS LYS |
| 272 | LEU LEU GLY GLY HIS LYS |
| 273 | LEU LEU ILE GLY HIS LYS |
| 274 | LEU LEU LEU GLY HIS LYS |
| 275 | LEU LEU MET GLY HIS LYS |
| 276 | LEU LEU PHE GLY HIS LYS |
| 277 | LEU LEU PRO GLY HIS LYS |
| 278 | LEU LEU TRP GLY HIS LYS |
| 279 | LEU LEU VAL GLY HIS LYS |
| 280 | LEU MET ALA GLY HIS LYS |
| 281 | LEU MET GLY GLY HIS LYS |
| 282 | LEU MET ILE GLY HIS LYS |
| 283 | LEU MET LEU GLY HIS LYS |
| 284 | LEU MET MET GLY HIS LYS |
| 285 | LEU MET PHE GLY HIS LYS |
| 286 | LEU MET PRO GLY HIS LYS |
| 287 | LEU MET TRP GLY HIS LYS |
| 288 | LEU MET VAL GLY HIS LYS |
| 289 | LEU PHE ALA GLY HIS LYS |
| 290 | LEU PHE GLY GLY HIS LYS |
| 291 | LEU PHE ILE GLY HIS LYS |
| 292 | LEU PHE LEU GLY HIS LYS |
| 293 | LEU PHE MET GLY HIS LYS |
| 294 | LEU PHE PHE GLY HIS LYS |
| 295 | LEU PHE PRO GLY HIS LYS |
| 296 | LEU PHE TRP GLY HIS LYS |
| 297 | LEU PHE VAL GLY HIS LYS |
| 298 | LEU PRO ALA GLY HIS LYS |
| 299 | LEU PRO GLY GLY HIS LYS |
| 300 | LEU PRO ILE GLY HIS LYS |
| 301 | LEU PRO LEU GLY HIS LYS |
| 302 | LEU PRO MET GLY HIS LYS |
| 303 | LEU PRO PHE GLY HIS LYS |
| 304 | LEU PRO PRO GLY HIS LYS |
| 305 | LEU PRO TRP GLY HIS LYS |
| 306 | LEU PRO VAL GLY HIS LYS |
| 307 | LEU TRP ALA GLY HIS LYS |

(continued)

| SEQ ID NO: | The structural formula of the cyclic peptide compound |
|---|---|
| 308 | LEU TRP GLY GLY HIS LYS |
| 309 | LEU TRP ILE GLY HIS LYS |
| 310 | LEU TRP LEU GLY HIS LYS |
| 311 | LEU TRP MET GLY HIS LYS |
| 312 | LEU TRP PHE GLY HIS LYS |
| 313 | LEU TRP PRO GLY HIS LYS |
| 314 | LEU TRP TRP GLY HIS LYS |
| 315 | LEU TRP VAL GLY HIS LYS |
| 316 | LEU VAL ALA GLY HIS LYS |
| 317 | LEU VAL GLY GLY HIS LYS |
| 318 | LEU VAL ILE GLY HIS LYS |
| 319 | LEU VAL LEU GLY HIS LYS |
| 320 | LEU VAL MET GLY HIS LYS |
| 321 | LEU VAL PHE GLY HIS LYS |
| 322 | LEU VAL PRO GLY HIS LYS |
| 323 | LEU VAL TRP GLY HIS LYS |
| 324 | LEU VAL VAL GLY HIS LYS |
| 325 | MET ALA ALA GLY HIS LYS |
| 326 | MET ALA GLY GLY HIS LYS |
| 327 | MET ALA ILE GLY HIS LYS |
| 328 | MET ALA LEU GLY HIS LYS |
| 329 | MET ALA MET GLY HIS LYS |
| 330 | MET ALA PHE GLY HIS LYS |
| 331 | MET ALA PRO GLY HIS LYS |
| 332 | MET ALA TRP GLY HIS LYS |
| 333 | MET ALA VAL GLY HIS LYS |
| 334 | MET GLY ALA GLY HIS LYS |
| 335 | MET GLY GLY GLY HIS LYS |
| 336 | MET GLY ILE GLY HIS LYS |
| 337 | MET GLY LEU GLY HIS LYS |
| 338 | MET GLY MET GLY HIS LYS |
| 339 | MET GLY PHE GLY HIS LYS |
| 340 | MET GLY PRO GLY HIS LYS |
| 341 | MET GLY TRP GLY HIS LYS |
| 342 | MET GLY VAL GLY HIS LYS |
| 343 | MET ILE ALA GLY HIS LYS |
| 344 | MET ILE GLY GLY HIS LYS |
| 345 | MET ILE ILE GLY HIS LYS |
| 346 | MET ILE LEU GLY HIS LYS |

(continued)

| SEQ ID NO: | The structural formula of the cyclic peptide compound |
|---|---|
| 347 | MET ILE MET GLY HIS LYS |
| 348 | MET ILE PHE GLY HIS LYS |
| 349 | MET ILE PRO GLY HIS LYS |
| 350 | MET ILE TRP GLY HIS LYS |
| 351 | MET ILE VAL GLY HIS LYS |
| 352 | MET LEU ALA GLY HIS LYS |
| 353 | MET LEU GLY GLY HIS LYS |
| 354 | MET LEU ILE GLY HIS LYS |
| 355 | MET LEU LEU GLY HIS LYS |
| 356 | MET LEU MET GLY HIS LYS |
| 357 | MET LEU PHE GLY HIS LYS |
| 358 | MET LEU PRO GLY HIS LYS |
| 359 | MET LEU TRP GLY HIS LYS |
| 360 | MET LEU VAL GLY HIS LYS |
| 361 | MET MET ALA GLY HIS LYS |
| 362 | MET MET GLY GLY HIS LYS |
| 363 | MET MET ILE GLY HIS LYS |
| 364 | MET MET LEU GLY HIS LYS |
| 365 | MET MET MET GLY HIS LYS |
| 366 | MET MET PHE GLY HIS LYS |
| 367 | MET MET PRO GLY HIS LYS |
| 368 | MET MET TRP GLY HIS LYS |
| 369 | MET MET VAL GLY HIS LYS |
| 370 | MET PHE ALA GLY HIS LYS |
| 371 | MET PHE GLY GLY HIS LYS |
| 372 | MET PHE ILE GLY HIS LYS |
| 373 | MET PHE LEU GLY HIS LYS |
| 374 | MET PHE MET GLY HIS LYS |
| 375 | MET PHE PHE GLY HIS LYS |
| 376 | MET PHE PRO GLY HIS LYS |
| 377 | MET PHE TRP GLY HIS LYS |
| 378 | MET PHE VAL GLY HIS LYS |
| 379 | MET PRO ALA GLY HIS LYS |
| 380 | MET PRO GLY GLY HIS LYS |
| 381 | MET PRO ILE GLY HIS LYS |
| 382 | MET PRO LEU GLY HIS LYS |
| 383 | MET PRO MET GLY HIS LYS |
| 384 | MET PRO PHE GLY HIS LYS |
| 385 | MET PRO PRO GLY HIS LYS |

(continued)

| SEQ ID NO: | The structural formula of the cyclic peptide compound |
|---|---|
| 386 | MET PRO TRP GLY HIS LYS |
| 387 | MET PRO VAL GLY HIS LYS |
| 388 | MET TRP ALA GLY HIS LYS |
| 389 | MET TRP GLY GLY HIS LYS |
| 390 | MET TRP ILE GLY HIS LYS |
| 391 | MET TRP LEU GLY HIS LYS |
| 392 | MET TRP MET GLY HIS LYS |
| 393 | MET TRP PHE GLY HIS LYS |
| 394 | MET TRP PRO GLY HIS LYS |
| 395 | MET TRP TRP GLY HIS LYS |
| 396 | MET TRP VAL GLY HIS LYS |
| 397 | MET VAL ALA GLY HIS LYS |
| 398 | MET VAL GLY GLY HIS LYS |
| 399 | MET VAL ILE GLY HIS LYS |
| 400 | MET VAL LEU GLY HIS LYS |
| 401 | MET VAL MET GLY HIS LYS |
| 402 | MET VAL PHE GLY HIS LYS |
| 403 | MET VAL PRO GLY HIS LYS |
| 404 | MET VAL TRP GLY HIS LYS |
| 405 | MET VAL VAL GLY HIS LYS |
| 406 | PHE ALA ALA GLY HIS LYS |
| 407 | PHE ALA GLY GLY HIS LYS |
| 408 | PHE ALA ILE GLY HIS LYS |
| 409 | PHE ALA LEU GLY HIS LYS |
| 410 | PHE ALA MET GLY HIS LYS |
| 411 | PHE ALA PHE GLY HIS LYS |
| 412 | PHE ALA PRO GLY HIS LYS |
| 413 | PHE ALA TRP GLY HIS LYS |
| 414 | PHE ALA VAL GLY HIS LYS |
| 415 | PHE GLY ALA GLY HIS LYS |
| 416 | PHE GLY GLY GLY HIS LYS |
| 417 | PHE GLY ILE GLY HIS LYS |
| 418 | PHE GLY LEU GLY HIS LYS |
| 419 | PHE GLY MET GLY HIS LYS |
| 420 | PHE GLY PHE GLY HIS LYS |
| 421 | PHE GLY PRO GLY HIS LYS |
| 422 | PHE GLY TRP GLY HIS LYS |
| 423 | PHE GLY VAL GLY HIS LYS |
| 424 | PHE ILE ALA GLY HIS LYS |

(continued)

| SEQ ID NO: | The structural formula of the cyclic peptide compound |
|---|---|
| 425 | PHE ILE GLY GLY HIS LYS |
| 426 | PHE ILE ILE GLY HIS LYS |
| 427 | PHE ILE LEU GLY HIS LYS |
| 428 | PHE ILE MET GLY HIS LYS |
| 429 | PHE ILE PHE GLY HIS LYS |
| 430 | PHE ILE PRO GLY HIS LYS |
| 431 | PHE ILE TRP GLY HIS LYS |
| 432 | PHE ILE VAL GLY HIS LYS |
| 433 | PHE LEU ALA GLY HIS LYS |
| 434 | PHE LEU GLY GLY HIS LYS |
| 435 | PHE LEU ILE GLY HIS LYS |
| 436 | PHE LEU LEU GLY HIS LYS |
| 437 | PHE LEU MET GLY HIS LYS |
| 438 | PHE LEU PHE GLY HIS LYS |
| 439 | PHE LEU PRO GLY HIS LYS |
| 440 | PHE LEU TRP GLY HIS LYS |
| 441 | PHE LEU VAL GLY HIS LYS |
| 442 | PHE MET ALA GLY HIS LYS |
| 443 | PHE MET GLY GLY HIS LYS |
| 444 | PHE MET ILE GLY HIS LYS |
| 445 | PHE MET LEU GLY HIS LYS |
| 446 | PHE MET MET GLY HIS LYS |
| 447 | PHE MET PHE GLY HIS LYS |
| 448 | PHE MET PRO GLY HIS LYS |
| 449 | PHE MET TRP GLY HIS LYS |
| 450 | PHE MET VAL GLY HIS LYS |
| 451 | PHE PHE ALA GLY HIS LYS |
| 452 | PHE PHE GLY GLY HIS LYS |
| 453 | PHE PHE ILE GLY HIS LYS |
| 454 | PHE PHE LEU GLY HIS LYS |
| 455 | PHE PHE MET GLY HIS LYS |
| 456 | PHE PHE PHE GLY HIS LYS |
| 457 | PHE PHE PRO GLY HIS LYS |
| 458 | PHE PHE TRP GLY HIS LYS |
| 459 | PHE PHE VAL GLY HIS LYS |
| 460 | PHE PRO ALA GLY HIS LYS |
| 461 | PHE PRO GLY GLY HIS LYS |
| 462 | PHE PRO ILE GLY HIS LYS |
| 463 | PHE PRO LEU GLY HIS LYS |

(continued)

| SEQ ID NO: | The structural formula of the cyclic peptide compound |
|---|---|
| 464 | PHE PRO MET GLY HIS LYS |
| 465 | PHE PRO PHE GLY HIS LYS |
| 466 | PHE PRO PRO GLY HIS LYS |
| 467 | PHE PRO TRP GLY HIS LYS |
| 468 | PHE PRO VAL GLY HIS LYS |
| 469 | PHE TRP ALA GLY HIS LYS |
| 470 | PHE TRP GLY GLY HIS LYS |
| 471 | PHE TRP ILE GLY HIS LYS |
| 472 | PHE TRP LEU GLY HIS LYS |
| 473 | PHE TRP MET GLY HIS LYS |
| 474 | PHE TRP PHE GLY HIS LYS |
| 475 | PHE TRP PRO GLY HIS LYS |
| 476 | PHE TRP TRP GLY HIS LYS |
| 477 | PHE TRP VAL GLY HIS LYS |
| 478 | PHE VAL ALA GLY HIS LYS |
| 479 | PHE VAL GLY GLY HIS LYS |
| 480 | PHE VAL ILE GLY HIS LYS |
| 481 | PHE VAL LEU GLY HIS LYS |
| 482 | PHE VAL MET GLY HIS LYS |
| 483 | PHE VAL PHE GLY HIS LYS |
| 484 | PHE VAL PRO GLY HIS LYS |
| 485 | PHE VAL TRP GLY HIS LYS |
| 486 | PHE VAL VAL GLY HIS LYS |
| 487 | PRO ALA ALA GLY HIS LYS |
| 488 | PRO ALA GLY GLY HIS LYS |
| 489 | PRO ALA ILE GLY HIS LYS |
| 490 | PRO ALA LEU GLY HIS LYS |
| 491 | PRO ALA MET GLY HIS LYS |
| 492 | PRO ALA PHE GLY HIS LYS |
| 493 | PRO ALA PRO GLY HIS LYS |
| 494 | PRO ALA TRP GLY HIS LYS |
| 495 | PRO ALA VAL GLY HIS LYS |
| 496 | PRO GLY ALA GLY HIS LYS |
| 497 | PRO GLY GLY GLY HIS LYS |
| 498 | PRO GLY ILE GLY HIS LYS |
| 499 | PRO GLY LEU GLY HIS LYS |
| 500 | PRO GLY MET GLY HIS LYS |
| 501 | PRO GLY PHE GLY HIS LYS |
| 502 | PRO GLY PRO GLY HIS LYS |

(continued)

| SEQ ID NO: | The structural formula of the cyclic peptide compound |
|---|---|
| 503 | PRO GLY TRP GLY HIS LYS |
| 504 | PRO GLY VAL GLY HIS LYS |
| 505 | PRO ILE ALA GLY HIS LYS |
| 506 | PRO ILE GLY GLY HIS LYS |
| 507 | PRO ILE ILE GLY HIS LYS |
| 508 | PRO ILE LEU GLY HIS LYS |
| 509 | PRO ILE MET GLY HIS LYS |
| 510 | PRO ILE PHE GLY HIS LYS |
| 511 | PRO ILE PRO GLY HIS LYS |
| 512 | PRO ILE TRP GLY HIS LYS |
| 513 | PRO ILE VAL GLY HIS LYS |
| 514 | PRO LEU ALA GLY HIS LYS |
| 515 | PRO LEU GLY GLY HIS LYS |
| 516 | PRO LEU ILE GLY HIS LYS |
| 517 | PRO LEU LEU GLY HIS LYS |
| 518 | PRO LEU MET GLY HIS LYS |
| 519 | PRO LEU PHE GLY HIS LYS |
| 520 | PRO LEU PRO GLY HIS LYS |
| 521 | PRO LEU TRP GLY HIS LYS |
| 522 | PRO LEU VAL GLY HIS LYS |
| 523 | PRO MET ALA GLY HIS LYS |
| 524 | PRO MET GLY GLY HIS LYS |
| 525 | PRO MET ILE GLY HIS LYS |
| 526 | PRO MET LEU GLY HIS LYS |
| 527 | PRO MET MET GLY HIS LYS |
| 528 | PRO MET PHE GLY HIS LYS |
| 529 | PRO MET PRO GLY HIS LYS |
| 530 | PRO MET TRP GLY HIS LYS |
| 531 | PRO MET VAL GLY HIS LYS |
| 532 | PRO PHE ALA GLY HIS LYS |
| 533 | PRO PHE GLY GLY HIS LYS |
| 534 | PRO PHE ILE GLY HIS LYS |
| 535 | PRO PHE LEU GLY HIS LYS |
| 536 | PRO PHE MET GLY HIS LYS |
| 537 | PRO PHE PHE GLY HIS LYS |
| 538 | PRO PHE PRO GLY HIS LYS |
| 539 | PRO PHE TRP GLY HIS LYS |
| 540 | PRO PHE VAL GLY HIS LYS |
| 541 | PRO PRO ALA GLY HIS LYS |

(continued)

| SEQ ID NO: | The structural formula of the cyclic peptide compound |
|---|---|
| 542 | PRO PRO GLY GLY HIS LYS |
| 543 | PRO PRO ILE GLY HIS LYS |
| 544 | PRO PRO LEU GLY HIS LYS |
| 545 | PRO PRO MET GLY HIS LYS |
| 546 | PRO PRO PHE GLY HIS LYS |
| 547 | PRO PRO PRO GLY HIS LYS |
| 548 | PRO PRO TRP GLY HIS LYS |
| 549 | PRO PRO VAL GLY HIS LYS |
| 550 | PRO TRP ALA GLY HIS LYS |
| 551 | PRO TRP GLY GLY HIS LYS |
| 552 | PRO TRP ILE GLY HIS LYS |
| 553 | PRO TRP LEU GLY HIS LYS |
| 554 | PRO TRP MET GLY HIS LYS |
| 555 | PRO TRP PHE GLY HIS LYS |
| 556 | PRO TRP PRO GLY HIS LYS |
| 557 | PRO TRP TRP GLY HIS LYS |
| 558 | PRO TRP VAL GLY HIS LYS |
| 559 | PRO VAL ALA GLY HIS LYS |
| 560 | PRO VAL GLY GLY HIS LYS |
| 561 | PRO VAL ILE GLY HIS LYS |
| 562 | PRO VAL LEU GLY HIS LYS |
| 563 | PRO VAL MET GLY HIS LYS |
| 564 | PRO VAL PHE GLY HIS LYS |
| 565 | PRO VAL PRO GLY HIS LYS |
| 566 | PRO VAL TRP GLY HIS LYS |
| 567 | PRO VAL VAL GLY HIS LYS |
| 568 | TRP ALA ALA GLY HIS LYS |
| 569 | TRP ALA GLY GLY HIS LYS |
| 570 | TRP ALA ILE GLY HIS LYS |
| 571 | TRP ALA LEU GLY HIS LYS |
| 572 | TRP ALA MET GLY HIS LYS |
| 573 | TRP ALA PHE GLY HIS LYS |
| 574 | TRP ALA PRO GLY HIS LYS |
| 575 | TRP ALA TRP GLY HIS LYS |
| 576 | TRP ALA VAL GLY HIS LYS |
| 577 | TRP GLY ALA GLY HIS LYS |
| 578 | TRP GLY GLY GLY HIS LYS |
| 579 | TRP GLY ILE GLY HIS LYS |
| 580 | TRP GLY LEU GLY HIS LYS |

(continued)

| SEQ ID NO: | The structural formula of the cyclic peptide compound |
|---|---|
| 581 | TRP GLY MET GLY HIS LYS |
| 582 | TRP GLY PHE GLY HIS LYS |
| 583 | TRP GLY PRO GLY HIS LYS |
| 584 | TRP GLY TRP GLY HIS LYS |
| 585 | TRP GLY VAL GLY HIS LYS |
| 586 | TRP ILE ALA GLY HIS LYS |
| 587 | TRP ILE GLY GLY HIS LYS |
| 588 | TRP ILE ILE GLY HIS LYS |
| 589 | TRP ILE LEU GLY HIS LYS |
| 590 | TRP ILE MET GLY HIS LYS |
| 591 | TRP ILE PHE GLY HIS LYS |
| 592 | TRP ILE PRO GLY HIS LYS |
| 593 | TRP ILE TRP GLY HIS LYS |
| 594 | TRP ILE VAL GLY HIS LYS |
| 595 | TRP LEU ALA GLY HIS LYS |
| 596 | TRP LEU GLY GLY HIS LYS |
| 597 | TRP LEU ILE GLY HIS LYS |
| 598 | TRP LEU LEU GLY HIS LYS |
| 599 | TRP LEU MET GLY HIS LYS |
| 600 | TRP LEU PHE GLY HIS LYS |
| 601 | TRP LEU PRO GLY HIS LYS |
| 602 | TRP LEU TRP GLY HIS LYS |
| 603 | TRP LEU VAL GLY HIS LYS |
| 604 | TRP MET ALA GLY HIS LYS |
| 605 | TRP MET GLY GLY HIS LYS |
| 606 | TRP MET ILE GLY HIS LYS |
| 607 | TRP MET LEU GLY HIS LYS |
| 608 | TRP MET MET GLY HIS LYS |
| 609 | TRP MET PHE GLY HIS LYS |
| 610 | TRP MET PRO GLY HIS LYS |
| 611 | TRP MET TRP GLY HIS LYS |
| 612 | TRP MET VAL GLY HIS LYS |
| 613 | TRP PHE ALA GLY HIS LYS |
| 614 | TRP PHE GLY GLY HIS LYS |
| 615 | TRP PHE ILE GLY HIS LYS |
| 616 | TRP PHE LEU GLY HIS LYS |
| 617 | TRP PHE MET GLY HIS LYS |
| 618 | TRP PHE PHE GLY HIS LYS |
| 619 | TRP PHE PRO GLY HIS LYS |

(continued)

| SEQ ID NO: | The structural formula of the cyclic peptide compound |
|---|---|
| 620 | TRP PHE TRP GLY HIS LYS |
| 621 | TRP PHE VAL GLY HIS LYS |
| 622 | TRP PRO ALA GLY HIS LYS |
| 623 | TRP PRO GLY GLY HIS LYS |
| 624 | TRP PRO ILE GLY HIS LYS |
| 625 | TRP PRO LEU GLY HIS LYS |
| 626 | TRP PRO MET GLY HIS LYS |
| 627 | TRP PRO PHE GLY HIS LYS |
| 628 | TRP PRO PRO GLY HIS LYS |
| 629 | TRP PRO TRP GLY HIS LYS |
| 630 | TRP PRO VAL GLY HIS LYS |
| 631 | TRP TRP ALA GLY HIS LYS |
| 632 | TRP TRP GLY GLY HIS LYS |
| 633 | TRP TRP ILE GLY HIS LYS |
| 634 | TRP TRP LEU GLY HIS LYS |
| 635 | TRP TRP MET GLY HIS LYS |
| 636 | TRP TRP PHE GLY HIS LYS |
| 637 | TRP TRP PRO GLY HIS LYS |
| 638 | TRP TRP TRP GLY HIS LYS |
| 639 | TRP TRP VAL GLY HIS LYS |
| 640 | TRP VAL ALA GLY HIS LYS |
| 641 | TRP VAL GLY GLY HIS LYS |
| 642 | TRP VAL ILE GLY HIS LYS |
| 643 | TRP VAL LEU GLY HIS LYS |
| 644 | TRP VAL MET GLY HIS LYS |
| 645 | TRP VAL PHE GLY HIS LYS |
| 646 | TRP VAL PRO GLY HIS LYS |
| 647 | TRP VAL TRP GLY HIS LYS |
| 648 | TRP VAL VAL GLY HIS LYS |
| 649 | VAL ALA ALA GLY HIS LYS |
| 650 | VAL ALA GLY GLY HIS LYS |
| 651 | VAL ALA ILE GLY HIS LYS |
| 652 | VAL ALA LEU GLY HIS LYS |
| 653 | VAL ALA MET GLY HIS LYS |
| 654 | VAL ALA PHE GLY HIS LYS |
| 655 | VAL ALA PRO GLY HIS LYS |
| 656 | VAL ALA TRP GLY HIS LYS |
| 657 | VAL ALA VAL GLY HIS LYS |
| 658 | VAL GLY ALA GLY HIS LYS |

(continued)

| SEQ ID NO: | The structural formula of the cyclic peptide compound |
|---|---|
| 659 | VAL GLY GLY GLY HIS LYS |
| 660 | VAL GLY ILE GLY HIS LYS |
| 661 | VAL GLY LEU GLY HIS LYS |
| 662 | VAL GLY MET GLY HIS LYS |
| 663 | VAL GLY PHE GLY HIS LYS |
| 664 | VAL GLY PRO GLY HIS LYS |
| 665 | VAL GLY TRP GLY HIS LYS |
| 666 | VAL GLY VAL GLY HIS LYS |
| 667 | VAL ILE ALA GLY HIS LYS |
| 668 | VAL ILE GLY GLY HIS LYS |
| 669 | VAL ILE ILE GLY HIS LYS |
| 670 | VAL ILE LEU GLY HIS LYS |
| 671 | VAL ILE MET GLY HIS LYS |
| 672 | VAL ILE PHE GLY HIS LYS |
| 673 | VAL ILE PRO GLY HIS LYS |
| 674 | VAL ILE TRP GLY HIS LYS |
| 675 | VAL ILE VAL GLY HIS LYS |
| 676 | VAL LEU ALA GLY HIS LYS |
| 677 | VAL LEU GLY GLY HIS LYS |
| 678 | VAL LEU ILE GLY HIS LYS |
| 679 | VAL LEU LEU GLY HIS LYS |
| 680 | VAL LEU MET GLY HIS LYS |
| 681 | VAL LEU PHE GLY HIS LYS |
| 682 | VAL LEU PRO GLY HIS LYS |
| 683 | VAL LEU TRP GLY HIS LYS |
| 684 | VAL LEU VAL GLY HIS LYS |
| 685 | VAL MET ALA GLY HIS LYS |
| 686 | VAL MET GLY GLY HIS LYS |
| 687 | VAL MET ILE GLY HIS LYS |
| 688 | VAL MET LEU GLY HIS LYS |
| 689 | VAL MET MET GLY HIS LYS |
| 690 | VAL MET PHE GLY HIS LYS |
| 691 | VAL MET PRO GLY HIS LYS |
| 692 | VAL MET TRP GLY HIS LYS |
| 693 | VAL MET VAL GLY HIS LYS |
| 694 | VAL PHE ALA GLY HIS LYS |
| 695 | VAL PHE GLY GLY HIS LYS |
| 696 | VAL PHE ILE GLY HIS LYS |
| 697 | VAL PHE LEU GLY HIS LYS |

(continued)

| SEQ ID NO: | The structural formula of the cyclic peptide compound |
|---|---|
| 698 | VAL PHE MET GLY HIS LYS |
| 699 | VAL PHE PHE GLY HIS LYS |
| 700 | VAL PHE PRO GLY HIS LYS |
| 701 | VAL PHE TRP GLY HIS LYS |
| 702 | VAL PHE VAL GLY HIS LYS |
| 703 | VAL PRO ALA GLY HIS LYS |
| 704 | VAL PRO GLY GLY HIS LYS |
| 705 | VAL PRO ILE GLY HIS LYS |
| 706 | VAL PRO LEU GLY HIS LYS |
| 707 | VAL PRO MET GLY HIS LYS |
| 708 | VAL PRO PHE GLY HIS LYS |
| 709 | VAL PRO PRO GLY HIS LYS |
| 710 | VAL PRO TRP GLY HIS LYS |
| 711 | VAL PRO VAL GLY HIS LYS |
| 712 | VAL TRP ALA GLY HIS LYS |
| 713 | VAL TRP GLY GLY HIS LYS |
| 714 | VAL TRP ILE GLY HIS LYS |
| 715 | VAL TRP LEU GLY HIS LYS |
| 716 | VAL TRP MET GLY HIS LYS |
| 717 | VAL TRP PHE GLY HIS LYS |
| 718 | VAL TRP PRO GLY HIS LYS |
| 719 | VAL TRP TRP GLY HIS LYS |
| 720 | VAL TRP VAL GLY HIS LYS |
| 721 | VAL VAL ALA GLY HIS LYS |
| 722 | VAL VAL GLY GLY HIS LYS |
| 723 | VAL VAL ILE GLY HIS LYS |
| 724 | VAL VAL LEU GLY HIS LYS |
| 725 | VAL VAL MET GLY HIS LYS |
| 726 | VAL VAL PHE GLY HIS LYS |
| 727 | VAL VAL PRO GLY HIS LYS |
| 728 | VAL VAL TRP GLY HIS LYS |
| 729 | VAL VAL VAL GLY HIS LYS |

In some embodiments, the structural formula of the cyclic peptide compound is as shown in Table B.

Table B: The preferred structural formula of the cyclic peptide compound (all amino acids are of L-configuration)

| SEQ ID NO: | The structural formula of the cyclic peptide compound |
|---|---|
| 52 | ALA PHE PRO GLY HIS LYS |
| 214 | ILE PHE PRO GLY HIS LYS |
| 414 | PHE ALA VAL GLY HIS LYS |

(continued)

| SEQ ID NO: | The structural formula of the cyclic peptide compound |
|---|---|
| 250 | LEU ALA PRO GLY HIS LYS |
| 297 | LEU PHE VAL GLY HIS LYS |
| 205 | ILE MET PRO GLY HIS LYS |
| 289 | LEU PHE ALA GLY HIS LYS |
| 291 | LEU PHE ILE GLY HIS LYS |
| 196 | ILE LEU PRO GLY HIS LYS |
| 232 | ILE TRP PRO GLY HIS LYS |
| 268 | LEU ILE PRO GLY HIS LYS |
| 292 | LEU PHE LEU GLY HIS LYS |
| 707 | VAL PRO MET GLY HIS LYS |
| 340 | MET GLY PRO GLY HIS LYS |
| 710 | VAL PRO TRP GLY HIS LYS |
| 293 | LEU PHE MET GLY HIS LYS |
| 169 | ILE ALA PRO GLY HIS LYS |
| 79 | ALA VAL PRO GLY HIS LYS |
| 421 | PHE GLY PRO GLY HIS LYS |
| 296 | LEU PHE TRP GLY HIS LYS |
| 46 | ALA PHE ALA GLY HIS LYS |
| 295 | LEU PHE PRO GLY HIS LYS |
| 25 | ALA ILE PRO GLY HIS LYS |
| 246 | LEU ALA ILE GLY HIS LYS |
| 286 | LEU MET PRO GLY HIS LYS |
| 304 | LEU PRO PRO GLY HIS LYS |
| 252 | LEU ALA VAL GLY HIS LYS |
| 277 | LEU LEU PRO GLY HIS LYS |
| 380 | MET PRO GLY GLY HIS LYS |
| 461 | PHE PRO GLY GLY HIS LYS |
| 160 | GLY VAL PRO GLY HIS LYS |
| 367 | MET MET PRO GLY HIS LYS |
| 358 | MET LEU PRO GLY HIS LYS |
| 349 | MET ILE PRO GLY HIS LYS |
| 62 | ALA PRO TRP GLY HIS LYS |
| 3 | ALA ALA ILE GLY HIS LYS |
| 4 | ALA ALA LEU GLY HIS LYS |
| 9 | ALA ALA VAL GLY HIS LYS |
| 84 | GLY ALA ILE GLY HIS LYS |
| 85 | GLY ALA LEU GLY HIS LYS |
| 88 | GLY ALA PRO GLY HIS LYS |
| 90 | GLY ALA VAL GLY HIS LYS |

(continued)

| SEQ ID NO: | The structural formula of the cyclic peptide compound |
|---|---|
| 165 | ILE ALA ILE GLY HIS LYS |
| 166 | ILE ALA LEU GLY HIS LYS |
| 168 | ILE ALA PHE GLY HIS LYS |
| 171 | ILE ALA VAL GLY HIS LYS |
| 245 | LEU ALA GLY GLY HIS LYS |
| 247 | LEU ALA LEU GLY HIS LYS |
| 248 | LEU ALA MET GLY HIS LYS |
| 249 | LEU ALA PHE GLY HIS LYS |
| 251 | LEU ALA TRP GLY HIS LYS |
| 325 | MET ALA ALA GLY HIS LYS |
| 327 | MET ALA ILE GLY HIS LYS |
| 328 | MET ALA LEU GLY HIS LYS |
| 331 | MET ALA PRO GLY HIS LYS |
| 333 | MET ALA VAL GLY HIS LYS |
| 406 | PHE ALA ALA GLY HIS LYS |
| 407 | PHE ALA GLY GLY HIS LYS |
| 408 | PHE ALA ILE GLY HIS LYS |
| 409 | PHE ALA LEU GLY HIS LYS |
| 412 | PHE ALA PRO GLY HIS LYS |
| 568 | TRP ALA ALA GLY HIS LYS |
| 569 | TRP ALA GLY GLY HIS LYS |
| 570 | TRP ALA ILE GLY HIS LYS |
| 571 | TRP ALA LEU GLY HIS LYS |
| 574 | TRP ALA PRO GLY HIS LYS |
| 576 | TRP ALA VAL GLY HIS LYS |
| 649 | VAL ALA ALA GLY HIS LYS |
| 650 | VAL ALA GLY GLY HIS LYS |
| 651 | VAL ALA ILE GLY HIS LYS |
| 652 | VAL ALA LEU GLY HIS LYS |
| 655 | VAL ALA PRO GLY HIS LYS |
| 657 | VAL ALA VAL GLY HIS LYS |

In some embodiments, the structural formula of the cyclic peptide compound is as shown in Table C.

Table C: The more preferred structural formula of the cyclic peptide compound (all amino acids are of L-configuration)

| SEQ ID NO: | The structural formula of the cyclic peptide compound |
|---|---|
| 4 | ALA ALA LEU GLY HIS LYS |
| 9 | ALA ALA VAL GLY HIS LYS |
| 25 | ALA ILE PRO GLY HIS LYS |
| 46 | ALA PHE ALA GLY HIS LYS |

(continued)

| SEQ ID NO: | The structural formula of the cyclic peptide compound |
|---|---|
| 52 | ALA PHE PRO GLY HIS LYS |
| 79 | ALA VAL PRO GLY HIS LYS |
| 85 | GLY ALA LEU GLY HIS LYS |
| 88 | GLY ALA PRO GLY HIS LYS |
| 160 | GLY VAL PRO GLY HIS LYS |
| 166 | ILE ALA LEU GLY HIS LYS |
| 169 | ILE ALA PRO GLY HIS LYS |
| 171 | ILE ALA VAL GLY HIS LYS |
| 196 | ILE LEU PRO GLY HIS LYS |
| 205 | ILE MET PRO GLY HIS LYS |
| 214 | ILE PHE PRO GLY HIS LYS |
| 232 | ILE TRP PRO GLY HIS LYS |
| 245 | LEU ALA GLY GLY HIS LYS |
| 246 | LEU ALA ILE GLY HIS LYS |
| 247 | LEU ALA LEU GLY HIS LYS |
| 249 | LEU ALA PHE GLY HIS LYS |
| 250 | LEU ALA PRO GLY HIS LYS |
| 252 | LEU ALA VAL GLY HIS LYS |
| 268 | LEU ILE PRO GLY HIS LYS |
| 286 | LEU MET PRO GLY HIS LYS |
| 289 | LEU PHE ALA GLY HIS LYS |
| 291 | LEU PHE ILE GLY HIS LYS |
| 292 | LEU PHE LEU GLY HIS LYS |
| 293 | LEU PHE MET GLY HIS LYS |
| 295 | LEU PHE PRO GLY HIS LYS |
| 296 | LEU PHE TRP GLY HIS LYS |
| 297 | LEU PHE VAL GLY HIS LYS |
| 304 | LEU PRO PRO GLY HIS LYS |
| 340 | MET GLY PRO GLY HIS LYS |
| 380 | MET PRO GLY GLY HIS LYS |
| 406 | PHE ALA ALA GLY HIS LYS |
| 409 | PHE ALA LEU GLY HIS LYS |
| 412 | PHE ALA PRO GLY HIS LYS |
| 414 | PHE ALA VAL GLY HIS LYS |
| 421 | PHE GLY PRO GLY HIS LYS |
| 461 | PHE PRO GLY GLY HIS LYS |
| 568 | TRP ALA ALA GLY HIS LYS |
| 574 | TRP ALA PRO GLY HIS LYS |
| 576 | TRP ALA VAL GLY HIS LYS |

(continued)

| SEQ ID NO: | The structural formula of the cyclic peptide compound |
|---|---|
| 649 | VAL ALA ALA GLY HIS LYS |
| 650 | VAL ALA GLY GLY HIS LYS |
| 652 | VAL ALA LEU GLY HIS LYS |
| 655 | VAL ALA PRO GLY HIS LYS |
| 707 | VAL PRO MET GLY HIS LYS |
| 710 | VAL PRO TRP GLY HIS LYS |

In some embodiments, the structural formula of the cyclic peptide compound is as shown in Table D.

Table D: The most preferred structural formula of the cyclic peptide compound (all amino acids are of L-configuration)

| SEQ ID NO: | The structural formula of the cyclic peptide compound |
|---|---|
| 414 | PHE ALA VAL GLY HIS LYS |
| 250 | LEU ALA PRO GLY HIS LYS |
| 205 | ILE MET PRO GLY HIS LYS |
| 292 | LEU PHE LEU GLY HIS LYS |
| 52 | ALA PHE PRO GLY HIS LYS |
| 214 | ILE PHE PRO GLY HIS LYS |
| 297 | LEU PHE VAL GLY HIS LYS |
| 289 | LEU PHE ALA GLY HIS LYS |
| 291 | LEU PHE ILE GLY HIS LYS |
| 196 | ILE LEU PRO GLY HIS LYS |
| 232 | ILE TRP PRO GLY HIS LYS |
| 268 | LEU ILE PRO GLY HIS LYS |
| 707 | VAL PRO MET GLY HIS LYS |
| 340 | MET GLY PRO GLY HIS LYS |
| 710 | VAL PRO TRP GLY HIS LYS |

The amino acid on the left side and the amino acid on the right side form a loop via a peptide bond in the structural formula of the cyclic peptide compound shown in Table A, Table B, Table C, and Table D.

In a second aspect of the invention, the present invention provides a pharmaceutical composition comprising the cyclic peptide compound or the stereoisomer thereof as previously described.

In some embodiments, the pharmaceutical composition further comprises at least one carrier and/or excipient.

In a third aspect of the invention, the present invention provides a cosmetic composition (such as a skin care product, a cosmetic, a hair growth solution, etc.) comprising the cyclic peptide compound or the stereoisomer thereof as previously described.

In some embodiments, the cosmetic composition further comprises a functional substance.

In some embodiments, the functional substance is one or more selected from the group consisting of skin conditioning agents, antioxidants, surfactants, humectants, preservatives, chelating agents, and flavor essences.

In a fourth aspect of the invention, the present invention provides use of the cyclic peptide compound or the stereoisomer thereof as previously described or the pharmaceutical composition as previously described in the manufacture of a medicament which is used for one or more selected from the following (1) - (7):

(1) delaying skin aging;
(2) reducing skin damage;
(3) accelerating skin wound healing;

(4) reducing hyperpigmentation;
(5) stimulating hair growth;
(6) improving the success rate of hair transplantation;
(7) preventing hair loss.

In a fifth aspect of the invention, the present invention provides the cyclic peptide compound or the stereoisomer thereof as previously described or the pharmaceutical composition as previously described for use in one or more uses selected from the following (1) - (7):

(1) delaying skin aging;
(2) reducing skin damage;
(3) accelerating skin wound healing;
(4) reducing hyperpigmentation;
(5) stimulating hair growth;
(6) improving the success rate of hair transplantation;
(7) preventing hair loss.

In a sixth aspect of the invention, the present invention provides a method selected from one or more of the following (1) - (7), the method comprising: administering to a subject in need thereof an effective amount of the cyclic peptide compound or the stereoisomer thereof as previously described or the pharmaceutical composition as previously described,

(1) a method for delaying skin aging;
(2) a method for reducing skin damage;
(3) a method for accelerating skin wound healing;
(4) a method for reducing hyperpigmentation;
(5) a method for stimulating hair growth;
(6) a method for improving the success rate of hair transplantation;
(7) a method for preventing hair loss.

In a seventh aspect of the invention, the present invention provides use of the cyclic peptide compound or the stereoisomer thereof as previously described or the cosmetic composition as previously described in one or more selected from the following (1) - (17), wherein the use is a non-therapeutic use,

(1) anti-aging of the skin;
(2) anti-wrinkling of the skin and reducing fine lines;
(3) improving mechanical properties of the skin: firmness, complexion, elasticity, and/or flexibility;
(4) increasing skin density (reconstitution effect);
(5) increasing skin volume (abundance effect);
(6) combating the appearance of stretch marks;
(7) reducing skin damage;
(8) accelerating skin wound healing;
(9) anti-spot;
(10) improving skin homogeneity and/or luster;
(11) reducing skin pigmentation;
(12) moisturizing;
(13) stimulating hair growth;
(14) improving the success rate of hair transplantation;
(15) preventing hair loss;
(16) topical cosmetology of eyelashes;
(17) increasing hair width, density, and/or length.

In an eighth aspect of the invention, the present invention provides a method selected from one or more of the following (1) - (17), the method comprising: administering to a subject in need thereof an effective amount of the cyclic peptide compound or the stereoisomer thereof as previously described or a cosmetic composition as previously described,

(1) a method for anti-aging of the skin;

(2) a method for anti-wrinkling of the skin and reducing fine lines;
(3) a method for improving mechanical properties of the skin: firmness, complexion, elasticity, and/or flexibility;
(4) a method for increasing skin density (reconstitution effect);
(5) a method for increasing skin volume (abundance effect);
(6) a method for combating the appearance of stretch marks;
(7) a method for reducing skin damage;
(8) a method for accelerating skin wound healing;
(9) a method for anti-spot;
(10) a method for improving skin homogeneity and/or luster;
(11) a method for reducing skin pigmentation;
(12) a method for moisturizing;
(13) a method for stimulating hair growth;
(14) a method for improving the success rate of hair transplantation;
(15) a method for preventing hair loss;
(16) a method for topical cosmetology of eyelashes;
(17) a method for increasing hair width, density, and/or length.

In a ninth aspect of the invention, the present invention provides use of the cyclic peptide compound or the stereoisomer thereof as previously described or the cosmetic composition as previously described in one or more selected from the following (1) - (17):

(1) anti-aging of the skin;
(2) anti-wrinkling of the skin and reducing fine lines;
(3) improving mechanical properties of the skin: firmness, complexion, elasticity, and/or flexibility;
(4) increasing skin density (reconstitution effect);
(5) increasing skin volume (abundance effect);
(6) combating the appearance of stretch marks;
(7) reducing skin damage;
(8) accelerating skin wound healing;
(9) anti-spot;
(10) improving skin homogeneity and/or luster;
(11) reducing skin pigmentation;
(12) moisturizing;
(13) stimulating hair growth;
(14) improving the success rate of hair transplantation;
(15) preventing hair loss;
(16) topical cosmetology of eyelashes;
(17) increasing hair width, density, and/or length.

## Beneficial Effects

1. The cyclic peptide compound of the present invention (e.g., the cyclic peptide compound in Table A, Table B, Table C, or Table D) or the stereoisomer thereof can achieve anti-wrinkling efficacy by significantly increasing Collagen I content.
2. The cyclic peptide compound of the present invention (e.g., the cyclic peptide compound in Table A, Table B, Table C, or Table D) or the stereoisomer thereof can significantly increase hyaluronic acid (HA) content, thereby achieving compactness efficacy.
3. The cyclic peptide compound of the present invention (e.g., the cyclic peptide compound in Table A, Table B, Table C, or Table D) or the stereoisomer thereof can significantly increase elastin content, thereby achieving compactness efficacy.
4. The cyclic peptide compound of the present invention (e.g., the cyclic peptide compound in Table A, Table B, Table C, or Table D) or the stereoisomer thereof can significantly increase the Collagen I content, the hyaluronic acid (HA) content and the elastin content, thereby achieving anti-wrinkling efficacy and compactness efficacy with excellent medical and cosmetic effects.
5. The cyclic peptide compound of the present invention (e.g., the cyclic peptide compound in Table A, Table B, Table C, or Table D) does not exhibit significant cytotoxicity (e.g. within the concentration range of 5 $\mu$M) and has a good

prospect for pharmaceutical and cosmetic applications.

6. The cyclic peptide compound of the present invention (e.g., the cyclic peptide compound in Table A, Table B, Table C, or Table D) or the stereoisomer thereof, when used as a medicament, has one or more beneficial effects selected from the following:

(1) delaying skin aging;
(2) reducing skin damage;
(3) accelerating skin wound healing;
(4) reducing hyperpigmentation;
(5) stimulating hair growth;
(6) improving the success rate of hair transplantation;
(7) preventing hair loss.

7. The cyclic peptide compound of the present invention (e.g., the cyclic peptide compound in Table A, Table B, Table C, or Table D) or the stereoisomer thereof, when used as a cosmetic for a non-therapeutic use, has one or more beneficial effects selected from the following:

(1) anti-aging of the skin;
(2) anti-wrinkling of the skin and reducing fine lines;
(3) improving mechanical properties of the skin: firmness, complexion, elasticity, and/or flexibility;
(4) increasing skin density (reconstitution effect);
(5) increasing skin volume (abundance effect);
(6) combating the appearance of stretch marks;
(7) reducing skin damage;
(8) accelerating skin wound healing;
(9) anti-spot;
(10) improving skin homogeneity and/or luster;
(11) reducing skin pigmentation;
(12) moisturizing;
(13) stimulating hair growth;
(14) improving the success rate of hair transplantation;
(15) preventing hair loss;
(16) topical cosmetology of eyelashes;
(17) increasing hair width, density, and/or length.

8. The cyclic peptide compound of the present invention (e.g., the cyclic peptide compound in Table A, Table B, Table C, or Table D) or the stereoisomer thereof, when used as a cosmetic, has one or more beneficial effects selected from the following:

(1) anti-aging of the skin;
(2) anti-wrinkling of the skin and reducing fine lines;
(3) improving mechanical properties of the skin: firmness, complexion, elasticity, and/or flexibility;
(4) increasing skin density (reconstitution effect);
(5) increasing skin volume (abundance effect);
(6) combating the appearance of stretch marks;
(7) reducing skin damage;
(8) accelerating skin wound healing;
(9) anti-spot;
(10) improving skin homogeneity and/or luster;
(11) reducing skin pigmentation;
(12) moisturizing;
(13) stimulating hair growth;
(14) improving the success rate of hair transplantation;
(15) preventing hair loss;
(16) topical cosmetology of eyelashes;
(17) increasing hair width, density, and/or length.

9. The efficacy of the cyclic peptide compound of the present invention (e.g., the cyclic peptide compound in Table A,

Table B, Table C, or Table D) or the stereoisomer thereof in the above eight aspects are comparable to or even better than that of GHK tripeptide.

## DETAILED DESCRIPTION OF EMBODIMENTS

Embodiments of the present invention will be described in detail below with reference to examples; however, those skilled in the art will understand that the following examples are only used to illustrate the present invention and should not be regarded as limiting the scope of the present invention. If no specific conditions are specified in the examples, experiments are carried out according to conventional conditions or conditions recommended by the manufacturer. If manufacturers of reagents or instruments used are not specified, the reagents or instruments are conventional products commercially available.

In the present invention, scientific and technical terms used herein have the meanings commonly understood by those skilled in the art unless otherwise indicated. Moreover, the terms and laboratory procedures as used herein related to the chemistry of proteins and nucleic acids, molecular biology, cell and tissue culture, microbiology, and immunology are commonly used terms and routine procedures in their respective fields. At the same time, for a better understanding of the present invention, definitions and explanations of relevant terms are provided below.

The term "stereoisomer" means that in a compound molecule of the same formula, atoms or substituents are linked to each other in the same order, but are in different spatial arrangements, belonging to an isomerism in organic chemistry.

The term "absent" means that the groups on both sides are directly linked. For example, if AA4 is absent in a cyclic peptide compound represented by formula I, the structural formula of the cyclic peptide compound represented by the formula I has become *AA1-AA2-GLY-HIS-LYS-AA3*.

The term "carrier" refers to a system that can alter the way a drug enters a person's body and distribute the drug in the body, control the release rate of the drug, and deliver the drug to the targeted organ. Drug carrier release and targeting systems can reduce drug degradation and loss, reduce side effects, and improve bioavailability. The macromolecular surfactants that can be used as carriers, due to their unique amphiphilic structure, can be self-assembled, forming various forms of aggregates, such as micelles, microemulsions, gels, liquid crystals, vesicles, etc., as preferred examples. These aggregates have the ability to encapsulate and load drug molecules and at the same time have good permeability to membranes, which can serve as excellent drug carriers.

The term "excipient" refers to an additive in a pharmaceutical formulation other than the main drug, which may also be referred to as an auxiliary material. The auxiliary material includes, but is not limited to: an ion exchanger, alumina, aluminum stearate, lecithin, serum proteins such as albumin prepared from human plasma, buffer substances such as phosphate, glycerol, sorbic acid, potassium sorbate, a mixture of partial glycerides of saturated vegetable fatty acids, water, salts or electrolytes such as protamine sulfate, disodium hydrogen phosphate, potassium hydrogen phosphate, sodium chloride, zinc salts, colloidal silica, magnesium trisilicate, polyvinylpyrrolidone, cellulose substances, polyethylene glycols, sodium carboxymethyl cellulose, polyacrylates, bee wax, and lanolin.

The term "skin conditioning agent" includes, but is not limited to, hydroquinone, kojic acid, arbutin, ellagic acid, azelaic acid, azelaionyldiglycine, L-ascorbic acid and derivatives thereof, licorice extract, *Malpighia emarginata* fruit extract, *Hydrangea macrophylla* extract, alkoxysalicylic acid, *Polygonum cuspidatum* extract, *Sophora japonica* flower extract, turmeric extract, mulberry bark extract, *Phyllanthus emblica* extract, Pea extract, aloin, etc.

The term "antioxidant" includes, but is not limited to, butylated hydroxytoluene, butylated hydroxyanisole, propyl gallate, octyl gallate, dodecanol gallate, tert-butyl hydroquinone, tocopherol, ascorbyl palmitate, isopropyl citrate (mixture), monoglyceride citrate, anoxomer, sodium sulfite, sodium bisulfite, sodium pyrosulfite, cysteine, erythorbic acid, thiourea, dilauryl thiomalonate, nordihydro-guaiaretic acid, thioctic acid, alpha-tocopheryl acetate, carotenoids, *Phyllanthus emblica* tannin, ascorbic acid, glutathione, coenzyme Q10, etc.

The term "surfactant" may be an anionic surfactant, a cationic surfactant, a nonionic surfactant, an ampholytic surfactant, a natural surfactant, a fluorine-containing surfactant, etc.

The term "humectant" includes, but is not limited to, glycerol, propylene glycol, butylene glycol, sorbitol, Sorbeth-20, amide humectants, glyceropolyether humectants, lactic acid and sodium lactate, sodium pyrrolidone carboxylate, hydroxyethyl urea, erythritol, glyceropolyether-5 lactate, sodium hyaluronate, saccharide isomers, D-panthenol, polyethylene glycol, diglycerol, methacryloyloxyethyl phosphorylcholine, etc.

The term "preservative" includes, but is not limited to, parabens and salts thereof, formaldehyde and formaldehyde donors, isothiazolinones, phenol preservatives, acid preservatives, halide preservatives, quaternary ammonium compounds, alcohols, commonly used compound preservatives and natural preservatives.

The term "chelating agent" can be selected from ethylenediaminetetraacetic acid and its sodium salt, glycine, citric acid, succinic acid, etc.

The term "flavor essence" may in particular be sandalwood flavor essence, jasmine flavor essence, rose flavor essence, sweet orange flavor essence, or vanilla flavor essence, etc. The flavor essence may also include essential oils, in

particular may be sour orange peel oil, bitter orange leaf/shoot oil, geranium oil, *Rosa damascena* flower oil, etc.

The term "treatment" generally refers to obtaining the desired pharmacological and/or physiological effects. The effect may be prophylactic on the basis of the complete or partial prevention of a disease or symptoms thereof; and/or the effect may be therapeutic on the basis of the partial or complete stabilization or cure of the disease and/or side effects due to the disease. As used herein, "treatment" covers any treatment of a disease in a patient, including: (a) prevention of a disease or symptom in a patient who is susceptible to the disease or symptom but has not been diagnosed with the disease; (b) suppressing a symptom of a disease, i.e., preventing the development thereof; or (c) relieving a symptom of a disease, i.e., causing the disease or symptom to resolve.

The term "subject" includes humans or non-human animals. In the present invention, the term "non-human animals" include all vertebrates, such as non-mammals (e.g., birds, amphibians, reptiles) and mammals such as non-human primates, livestock and/or domesticated animals (e.g., sheep, dogs, cats, cows, pigs, etc.).

The term "an effective amount" refers to an amount effective, at dosages and for periods of time necessary, to achieve a desired effect. The "effective amount" of the present invention may vary depending on factors such as the individual's skin/hair state, age, sex, and body weight.

In the present invention, 20 conventional amino acids and their abbreviations follow the conventional usage. See Immunology-A Synthesis (the 2nd ed., E.S. Golub and D.R. Gren, Eds., Sinauer Associates, Sunderland, Mass. (1991), which is incorporated herein by reference.

As is well known to those skilled in the art, the term "amino acid residue" refers to the incomplete amino acid structure with an amino terminus (N-terminus) and a carboxy terminus (C-terminus) remained after an amino acid loses one hydrogen on its amino group and one hydroxyl group on its carboxyl group.

In the cyclic peptide compounds of the invention (including but not limited to the cyclic peptide compounds in Table A, Table B, Table C, and Table D), from left to right means from N-terminus to C-terminus by default, and the N-terminus on the left side is connected to C-terminus on the right side to form an NC cyclic peptide, unless otherwise specified.

In addition, the abbreviations in the examples have conventional meanings well known in the art, and are listed below by the way of illustration:

AcOH, Acetic Acid
ACN, Acetonitrile
DCM, Dichloromethane (methylene chloride)
DMF, N,N-Dimethylformamide
DIEA, N,N-Diisopropylethylamine
Fmoc, 9-fluorenylmethyloxycarbonyl
HOBT, 1-Hydroxybenzotriazole
Piperidine, piperidine
PyBOP, Benzotriazol-1-yl-oxytripyrrolidinophosphonium hexafluorophosphate
Resin, resin
TBTU, O-(Benzotriazol-1-yl)-N,N,N',N'-tetramethyluronium tetrafluoroborate
TFA, Trifluoroacetic acid
TFE, 2,2,2-Trifluoroethanol
TIS, Triisopropylsilane

The present invention will be further explained below in connection with specific examples, which are in no way to be construed as limiting the present invention. In addition, raw materials or reagents can be commercially available unless otherwise stated.

## Example 1: Synthesis and Identification of Cyclic Peptide Compounds

1. Synthesis of the Cyclic Peptide Compounds

Taking the cyclic peptide compound as set forth in SEQ ID NO: 52 as an example, condensation of the amino acids from C-terminus to N-terminus (from right to left) was performed sequentially by starting with lysine.

Step I. Solid-phase synthesis

Fmoc-Lys(Boc)-2-Cl Resin (S = 0.32 mmol/g) was weighed, placed in a glass reaction column, and swollen with DCM for 30 min. The DCM was removed under reduced pressure. 20% piperidine/DMF solution was added to react for 20 min so as to remove the protecting group Fmoc. The solution was removed under reduced pressure. The resin was washed

with DMF five times. Sampling and colour development test were performed. A solid-phase resin with Lys attached (via attachment to the C-terminus of Lys) was obtained.

Fmoc-His(Trt)-OH was weighed and added into the resin, and dissolved with DMF. DIEA and TBTU were added, and the mixture was stirred for 60 min. The solution was removed under reduced pressure. The resin was washed with DMF five times. Sampling and colour development test were performed. A solid-phase resin with Lys-His attached (from C-terminus to N-terminus) was obtained.

The process similar to that described above was repeated. Gly, Pro, Phe, and Ala were sequentially coupled. A solid-phase resin with the polypeptide Lys-His-Gly-Pro-Phe-Ala attached (from C-terminus to N-terminus) was finally obtained, i.e., the peptide resin.

Then, the resin was sequentially washed with DMF, DCM, and methanol three times, and was dried to constant weight.

Step II. Cleavage of the peptide resin

Preparation of cleavage reagent: Based on a ratio of 1 g of the peptide resin to 10 mL ± 2 mL of cleavage reagents (AcOH : TFE : DCM = 1 : 2 : 7, volume ratio), the usage amounts of the cleavage reagents were calculated. The specific steps were as follows: the desired cleavage reagents DCM, TFE and AcOH were sequentially added to a cleavage reaction flask, and the temperature of the cleavage reagents were controlled between 0-10°C; the cleavage reagents were added to the peptide resin with stirring, and the temperature of the system was allowed to be stabilized; after the reaction was stirred at a controlled temperature of 25-30°C for 2 hours, the cleavage solution was filtered off.

Step III. Dealing with the reaction

The cleavage solution obtained in the Step II was put into a TBTU/DIEA stirring reaction. After the reaction was completed, the solvent was evaporated off on a rotary evaporator. Then, based on a ratio of 1 g of the peptide resin to 10 ml ± 2 ml of the reagents to be mixed (TFA : $H_2O$ : TIS = 95 : 2.5 : 2.5, volume ratio), the usage amounts of the reagents to be mixed were calculated. Afterwards, the desired reagents $H_2O$, TFA, and TIS were sequentially added to the reaction flask. After the reaction was stirred for 2 hours, the product was precipitated with ice-cold diethyl ether in an amount 5 times the volume of the concentrated solution. The precipitate was filtered out and was dried under reduced pressure at room temperature to give a crude product.

Step IV. Lyophilization and purification

The crude product was ground into a fine powder. Purified water was prepared, and the ground crude product was added slowly with stirring. An acetonitrile-water solution was added dropwise simultaneously. After the addition and dissolution of the crude product were completed, the solution was filtered through a 0.45 μm microporous filter membrane. The crude product was purified using a C-18 packing preparative column. Mobile phase A was 0.1% TFA in water; and mobile phase B was 0.1% TFA in acetonitrile. Separation and purification were performed at ambient temperature with an appropriate gradient. The target products were collected, analyzed and detected, and classified. The purity requirement was ≥ 95%. Unqualified target product was collected and further separated and purified using an appropriate gradient. The qualified main peak was freeze-dried under reduced pressure to give the refined polypeptide (SEQ ID NO: 52) in a powder form.

Other cyclic peptide compounds of the present application can be prepared by those skilled in the art by reference to the synthesis procedures described above.

2. Identification of the Cyclic Peptide Compounds

The refined polypeptide prepared above was weighed, dissolved in a mixed solution of water and acetonitrile until the solution became clear, filtered and then loaded for detection. Detection conditions of high performance liquid chromatography: C18 column, 4.6 μm × 250 mm; mobile phase A: 0.1% TFA in acetonitrile, mobile phase B: 0.1% TFA in water; gradient conditions: 25% A and 75% B at 0.01 min→ 50% A and 50% B at 25 min → 100% A at 25.01 min → stop at 30 min; detection wavelength: 220 nm; flow rate: 1.0 ml/min; column temperature: 25°C; injection volume: 10 μL.

The detector for mass spectrometry was Agilent-6125B, using an electrospray ion source (ESI), with positive ion scanning; the voltage of mass spectrometry: +4.5 kv; the voltage of detector : 1.5 kv; the flow rate of sprayer: 1.5 L/min; CDL: -20.0 v; CDL temperature: 250°C; heating module temperature: 200°C; mobile phase: 50% water/50% acetonitrile; the flow rate of mobile phase: 0.2 mL/min.

Mass spectral data of some of the exemplary cyclic peptide compounds are shown in Table 1 below.

Table 1: Mass spectral data of the exemplary cyclic peptide compounds

| SEQ ID NO: | Theoretical MS value $[M+H]^+$ | Measured MS value $[M+H]^+$ |
|---|---|---|
| 52 | 638.7 | 639.0 |
| 214 | 680.8 | 680.8 |
| 414 | 640.8 | 640.6 |
| 250 | 604.7 | 604.8 |
| 297 | 682.8 | 682.8 |
| 205 | 664.8 | 665.0 |
| 289 | 654.8 | 654.4 |
| 291 | 696.9 | 697.0 |
| 196 | 646.8 | 646.3 |
| 232 | 719.9 | 719.4 |
| 268 | 646.8 | 646.5 |
| 292 | 696.9 | 696.5 |
| 707 | 650.8 | 650.5 |
| 340 | 608.7 | 608.4 |
| 710 | 705.8 | 705.4 |

As shown in Table 1, the desired cyclic peptide compounds can be successfully obtained by the preparation method of the present invention.

## Example 2: Biological Activity Test of the Cyclic Peptide Compounds

1. Test Methods

A. Fibroblast cytotoxicity test

Test materials: Fibroblasts of human origin (Guangdong Boxi Biotechnology Co., Ltd., Lot No. Fb20081902), DMEM culture medium (Gibco, 23042301, expiration date on 230522), PBS (Solarbio, P1010), MTT (Sigma, M5655), DMSO (Sigma, D4540-1L), $CO_2$ incubator (Thermo, 150I), super-clean bench (AIRTECH, SW-CJ-1F), inverted microscope (Olympus, CKX53).

Test methods:

1) Cell inoculation: After cell resuscitation, the cells were inoculated into 96-well plates when the plating rate reached around 60%, and were incubated overnight in an incubator (37°C, 5% $CO_2$).
2) Test grouping: The blank group, solvent control group, positive control group, and sample group were set for the test. In the sample group, 7 concentration gradients were set for each sample and 3 duplicate wells were set for each concentration gradient.
3) Formulation of solutions: Sample working solutions at different concentrations were formulated based on the test concentrations of 0.0781 μM, 0.1563 μM, 0.3125 μM, 0.625 μM, 1.25 μM, 2.5 μM, and 5 μM.
4) Administration: Administration was performed when the cell plating rate in the 96-well plates reached 50% to 60%. 200 μL of culture medium per well was added for the solvent control group; 200 μL of culture medium containing 10% DMSO per well was added for the positive control group; 200 μL of culture medium containing the sample at a corresponding concentration per well was added for the sample group; The blank group had no cell inoculation, for which only 200 μL of cell culture medium was added. After the administration was complete, the 96-well plates were incubated in an incubator (37°C, 5% CO2) for 24 h.
5) Detection: After 24 h of cell incubation and culture, the supernatant was discarded, and MTT working solution (0.5 mg/mL) was added. Incubation was performed at 37°C for 4 h in the dark. After the incubation was completed, the supernatant was discarded and 150 μL DMSO was added to each well. The OD value was read at 490 nm.
6) Calculation of relative viability of cells: Calculation was performed according to the formula below:

$$\text{Relative viability of cells} = \frac{\text{Sample well OD} - \text{Blank well OD}}{\text{Solvent control well OD} - \text{Blank well OD}} \times 100\%.$$

The detection results of some of the exemplary cyclic peptide compounds are shown in Table 2.

Table 2: The detection results of the exemplary cyclic peptide compounds

| | SE Q ID NO: | Concentration (μM) | | | | | | | Positiv e control |
|---|---|---|---|---|---|---|---|---|---|
| | | 0.0781 | 0.1563 | 0.3125 | 0.625 | 1.25 | 2.5 | 5 | 10% DMSO |
| Cyclic pep-tide com-pound s | 52 | 96.35% | 96.02% | 96.69% | 96.69% | 96.08% | 94.03% | 93.26% | 0.61% |
| | 214 | 99.39% | 98.67% | 98.40% | 98.67% | 97.51% | 97.51% | 95.36% | 0.61% |
| | 414 | 106.03 % | 105.69 % | 105.11 % | 104.20 % | 104.20 % | 102.07 % | 101.55 % | 2.07% |
| | 250 | 118.62 % | 117.01 % | 116.72 % | 114.43 % | 114.02 % | 114.60 % | 114.89 % | 2.07% |
| | 297 | 103.91 % | 102.94 % | 102.84 % | 102.78 % | 101.66 % | 100.59 % | 100.11 % | 0.54% |
| | 205 | 104.12 % | 104.87 % | 102.41 % | 102.03 % | 99.95% | 99.41% | 99.14% | 0.54% |
| | 289 | 98.48% | 96.48% | 95.25% | 95.39% | 95.91% | 95.01% | 94.87% | 0.43% |
| | 291 | 97.96% | 97.15% | 97.48% | 97.05% | 96.58% | 94.20% | 94.49% | 0.43% |
| | 196 | 101.92 % | 101.21 % | 102.58 % | 101.26 % | 100.40 % | 101.47 % | 99.85% | 0.30% |
| | 232 | 100.44 % | 97.64% | 93.46% | 94.05% | 93.90% | 91.59% | 91.10% | 1.87% |
| | 268 | 97.22% | 93.85% | 94.77% | 91.67% | 91.81% | 90.66% | 90.38% | 1.62% |
| | 292 | 107.68 % | 107.63 % | 105.05 % | 102.37 % | 102.07 % | 102.88 % | 102.88 % | 0.30% |
| | 707 | 102.27 % | 98.38% | 97.64% | 97.83% | 93.62% | 92.78% | 91.58% | 1.62% |
| | 340 | 98.04% | 98.71% | 97.29% | 96.66% | 94.99% | 94.28% | 91.65% | 1.17% |
| | 710 | 97.66% | 97.91% | 97.54% | 96.66% | 96.20% | 95.37% | 95.82% | 0.61% |

As shown in Table 2, in the fibroblast toxicity test experiment, none of the cyclic peptide compounds exhibited significant cytotoxicity within the concentration range of 5 μM, with good prospect for pharmaceutical and cosmetic applications.

B. Collagen I (type I collagen) elevation effect in fibroblasts, hyaluronic acid (HA) elevation effect in fibroblasts, and elastin (ELN) elevation effect in fibroblasts

Test materials: Fibroblasts of human origin (Guangdong Boxi Biotechnology Co., Ltd., Lot No. Fb20081902), DMEM culture medium (Gibco, 23042301, expiration date 230522), PBS (Solarbio, P1010), TGF-β1 (Peprotech, 230504), Collagen I ELISA Kit (Cusabio, B21025824), Elastin ELISA kit (Abcam, GR3446070-2), HA ELISA kit (RD, P325066). CO2 incubator (Thermo, 1501), super-clean bench (AIRTECH, SW-CJ-1F), microplate reader (BioTek, Epoch).

Test methods:

1) Inoculation: After cell resuscitation, the cells were inoculated into 6-well plates when the plating rate reached around 60%, and were incubated overnight in an incubator (37°C, 5% $CO_2$).
2) Formulation of solutions: The cyclic peptide compounds in the sample group, PC2 (Biotinoyl-GHK,

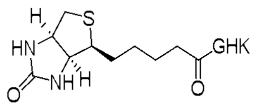

) and PC3

( 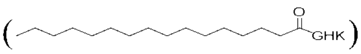 )

in the positive reference group were all at the concentration of 1 μM, and the test substance working solutions were formulated. Only the cell culture medium was added for the blank control group (BC).

3) Administration: When the cell plating rate in the 6-well plates reached 40% to 60%, administration was performed at 2 mL per well. 3 duplicate wells were set for each group. Incubation was performed in an incubator (37°C, 5% $CO_2$) for 24 h.

4) ELISA assay: The ELISA assay was performed according to the instructions of the ELISA kit, with the detection indicators being Collagen I elevation effect in the fibroblasts, HA elevation effect in the fibroblasts, and elastin elevation effect in the fibroblasts.

The activity test data of some of the exemplary cyclic peptide compounds are shown in Table 3A, Table 3B, and Table 3C below.

Table 3A: Data of the Collagen I elevation effect in the fibroblasts for some of the exemplary cyclic peptide compounds

| Blank control/SEQ ID NO: | Collagen I elevation effect in the fibroblasts |
|---|---|
| Blank control (BC) | 35.6 |
| 414 | 40.54 |
| 250 | 55.85 |
| 205 | 49.25 |
| 214 | 42.51 |
| 297 | 52.28 |
| 289 | 41 |
| 232 | 67.93 |
| 268 | 61.46 |
| 707 | 66.21 |
| 340 | 90.07 |
| 710 | 110.87 |

As shown in Table 3A, compared to the BC, the exemplary cyclic peptide compounds of the present invention, such as the cyclic peptide compounds as set forth in SEQ ID NOs: 214, 414, 250, 297, 205, 289, 232, 268, 707, 340, and 710 result in a significant increase in the type I collagen (Collagen I) content, demonstrating that the cyclic peptide compounds of the present invention can achieve **anti-wrinkling efficacy** by increasing the Collagen I content.

Table 3B: Data of the HA elevation effect in the fibroblasts for some of the exemplary cyclic peptide compounds

| Blank control/SEQ ID NO: | HA elevation effect in the fibroblasts |
|---|---|
| Blank control (BC) | 1402.44 |
| 414 | 1926.61 |
| 250 | 2142.56 |
| 205 | 2641.21 |
| 292 | 2635.98 |
| 52 | 2900.83 |

(continued)

| Blank control/SEQ ID NO: | HA elevation effect in the fibroblasts |
|---|---|
| 214 | 1965.3 |
| 297 | 2212.6 |
| 289 | 1978.48 |
| 291 | 2477.91 |
| 196 | 2305.13 |

As shown in Table 3B, compared to the BC, the exemplary cyclic peptide compounds of the present invention, such as the cyclic peptide compounds as set forth in SEQ ID NOs: 52, 250, 297, 205, 291, 196, 292, 414, 214, and 289 result in a significant increase in the hyaluronic acid (HA) content, demonstrating that the cyclic peptide compounds of the present invention can achieve **compactness efficacy** by increasing the HA content.

Table 3C: Data of the elastin elevation effect in the fibroblasts for some of the exemplary cyclic peptide compounds

| Blank control/SEQ ID NO: | Elastin elevation effect in the fibroblasts |
|---|---|
| Blank control (BC) | 756.52 |
| 414 | 1124.58 |
| 250 | 1051.64 |
| 205 | 1074.17 |
| 292 | 1278.99 |
| 52 | 1043.27 |
| 214 | 1050.82 |
| 297 | 923.52 |
| 289 | 1034.59 |
| 291 | 1177.19 |
| 196 | 1175.6 |
| 232 | 1001.84 |
| 340 | 1304.87 |
| 710 | 1207.29 |

As shown in Table 3C, compared to the BC, the exemplary cyclic peptide compounds of the present invention, such as the cyclic peptide compounds as set forth in SEQ ID NOs: 52, 250, 297, 205, 291, 196, 292, 414, 214, 289, 232, 340, and 710 result in a significant increase in the elastin content, demonstrating that the cyclic peptide compounds of the present invention can achieve **compactness efficacy** by increasing the elastin content.

In addition, as shown in Table 3A, Table 3B, and Table 3C, compared to the BC, the exemplary cyclic peptide compounds of the present invention, such as the cyclic peptide compounds as set forth in SEQ ID NOs: 414, 250, 205, and 292, result in a significant increase in the type I collagen (Collagen I) content, the hyaluronic acid (HA) content, the elastin content, demonstrating that the cyclic peptide compounds of the present invention can achieve the **compactness efficacy and anti-wrinkling efficacy** by increasing the Collagen I content, the HA content and the elastin content, with excellent medical and cosmetic effects.

## Claims

**1.** A cyclic peptide compound represented by formula I, or a stereoisomer thereof,

*AA1-AA2-GLY-HIS-LYS-AA4-AA3* Formula I

**characterized in that**:

AA1 is an amino acid residue;
AA2 is absent or is an amino acid residue;
AA3 is an amino acid residue;
AA4 is absent or is an amino acid residue;
* indicates that the N-terminus and the C-terminus of formula I are linked to form a cyclic peptide;
the amino acid is a natural amino acid or a non-natural amino acid;
the amino acid is of D-configuration or L-configuration, preferably of L-configuration.

2. The cyclic peptide compound or the stereoisomer thereof of claim 1, **characterized in that** the amino acid is a natural amino acid;

preferably, the amino acid is a hydrophobic natural amino acid;
more preferably, the amino acid is selected from the group consisting of alanine, glycine, isoleucine, leucine, methionine, phenylalanine, proline, tryptophan, and valine.

3. The cyclic peptide compound or the stereoisomer thereof of claim 1 or 2, **characterized in that** AA1 is selected from the group consisting of a phenylalanine residue, an alanine residue, a methionine residue, a leucine residue, a tryptophan residue, an isoleucine residue, a proline residue, a glycine residue, and a valine residue;

preferably, AA1 is selected from the group consisting of an alanine residue, a methionine residue, a phenylalanine residue, a leucine residue, a tryptophan residue, an isoleucine residue, a proline residue, and a glycine residue;
more preferably, AA1 is selected from the group consisting of a methionine residue, an alanine residue, a phenylalanine residue, a glycine residue, and a proline residue.

4. The cyclic peptide compound or the stereoisomer thereof of any one of claims 1-3, **characterized in that** AA2 is an amino acid residue;

preferably, AA2 is selected from the group consisting of a phenylalanine residue, an alanine residue, a methionine residue, a leucine residue, a tryptophan residue, an isoleucine residue, a proline residue, a glycine residue, and a valine residue;
preferably, AA2 is selected from the group consisting of a valine residue, a proline residue, a leucine residue, an alanine residue, an isoleucine residue, a methionine residue, and a tryptophan residue;
more preferably, AA2 is selected from the group consisting of a proline residue, a leucine residue, a valine residue, an isoleucine residue, and a tryptophan residue.

5. The cyclic peptide compound or the stereoisomer thereof of any one of claims 1-4, **characterized in that** AA3 is selected from the group consisting of a phenylalanine residue, an alanine residue, a methionine residue, a leucine residue, a tryptophan residue, an isoleucine residue, a proline residue, a glycine residue, and a valine residue;

preferably, AA3 is selected from the group consisting of a phenylalanine residue, an alanine residue, a methionine residue, a leucine residue, a tryptophan residue, an isoleucine residue, a glycine residue, and a valine residue;
preferably, AA3 is selected from the group consisting of a phenylalanine residue, an alanine residue, a methionine residue, a leucine residue, an isoleucine residue, and a valine residue;
more preferably, AA3 is selected from the group consisting of an isoleucine residue, a leucine residue, a phenylalanine residue, a methionine residue, and a valine residue.

6. The cyclic peptide compound or the stereoisomer thereof of any one of claims 1-5, **characterized in that** AA4 is absent.

7. The cyclic peptide compound or the stereoisomer thereof of any one of claims 1-6, **characterized in that** the cyclic peptide compound has a structure represented by formula I-1,

*AA3-AA1-AA2-GLY-HIS-LYS*    Formula I-1

wherein:

AA1 is selected from the group consisting of a phenylalanine residue, an alanine residue, a methionine residue, a leucine residue, a tryptophan residue, an isoleucine residue, a proline residue, a glycine residue, and a valine residue;
preferably, AA1 is selected from the group consisting of a phenylalanine residue, an alanine residue, a methionine residue, a leucine residue, a tryptophan residue, an isoleucine residue, a proline residue, and a glycine residue; and/or
AA2 is selected from the group consisting of a phenylalanine residue, an alanine residue, a methionine residue, a leucine residue, a tryptophan residue, an isoleucine residue, a proline residue, a glycine residue, and a valine residue;
preferably, AA2 is selected from the group consisting of an alanine residue, a methionine residue, a leucine residue, a tryptophan residue, an isoleucine residue, a proline residue, and a valine residue;
and/or
AA3 is selected from the group consisting of a phenylalanine residue, an alanine residue, a methionine residue, a leucine residue, a tryptophan residue, an isoleucine residue, a proline residue, a glycine residue, and a valine residue;
preferably, AA3 is selected from the group consisting of a phenylalanine residue, an alanine residue, a methionine residue, a leucine residue, a tryptophan residue, an isoleucine residue, a glycine residue, and a valine residue;
more preferably, AA3 is selected from the group consisting of a phenylalanine residue, an alanine residue, a methionine residue, a leucine residue, an isoleucine residue, and a valine residue;
* indicates that the N-terminus and the C-terminus of the formula I-1 are linked to form a cyclic peptide;
the amino acid is of D-configuration or L-configuration, preferably of L-configuration.

**8.** The cyclic peptide compound or the stereoisomer thereof of claim 7, **characterized in that** preferably AA1 is selected from the group consisting of a methionine residue, an alanine residue, a phenylalanine residue, a glycine residue, and a proline residue; and/or

preferably AA2 is selected from the group consisting of a proline residue, a leucine residue, a valine residue, an isoleucine residue, and a tryptophan residue; and/or
preferably, AA3 is selected from the group consisting of an isoleucine residue, a leucine residue, a phenylalanine residue, a methionine residue, and a valine residue.

**9.** The cyclic peptide compound or the stereoisomer thereof of any one of claims 1-8, **characterized in that** the structural formula of the cyclic peptide compound is as shown in Table A;

preferably, the structural formula of the cyclic peptide compound is as shown in Table B;
more preferably, the structural formula of the cyclic peptide compound is as shown in Table C;
most preferably, the structural formula of the cyclic peptide compound is as shown in Table D.

**10.** A pharmaceutical composition comprising the cyclic peptide compound or the stereoisomer thereof of any one of claims 1-9;
optionally, further comprising at least one carrier and/or excipient.

**11.** A cosmetic composition comprising the cyclic peptide compound or the stereoisomer thereof of any one of claims 1-9;

optionally, further comprising a functional substance;
preferably, the functional substance being one or more selected from the group consisting of skin conditioning agents, antioxidants, surfactants, humectants, preservatives, chelating agents, and flavor essences.

**12.** Use of the cyclic peptide compound or the stereoisomer thereof of any one of claims 1-9 or the pharmaceutical composition of claim 10 in the manufacture of a medicament which is used for one or more selected from the following (1) - (7):

(1) delaying skin aging;
(2) reducing skin damage;
(3) accelerating skin wound healing;
(4) reducing hyperpigmentation;
(5) stimulating hair growth;
(6) improving the success rate of hair transplantation;

(7) preventing hair loss.

**13.** Use of the cyclic peptide compound or the stereoisomer thereof of any one of claims 1-9 or the cosmetic composition of claim 11 in one or more selected from the following (1) - (17):

(1) anti-aging of the skin;
(2) anti-wrinkling of the skin and reducing fine lines;
(3) improving mechanical properties of the skin: firmness, complexion, elasticity, and/or flexibility;
(4) increasing skin density;
(5) increasing skin volume;
(6) combating the appearance of stretch marks;
(7) reducing skin damage;
(8) accelerating skin wound healing;
(9) anti-spot;
(10) improving skin homogeneity and/or luster;
(11) reducing skin pigmentation;
(12) moisturizing;
(13) stimulating hair growth;
(14) improving the success rate of hair transplantation;
(15) preventing hair loss;
(16) topical cosmetology of eyelashes;
(17) increasing hair width, density, and/or length.

| INTERNATIONAL SEARCH REPORT | International application No. PCT/CN2024/120959 |
|---|---|

**A. CLASSIFICATION OF SUBJECT MATTER**

C07K5/12(2006.01)i; C07K 7/64(2006.01)i; A61K8/64(2006.01)i; A61Q19/00(2006.01)i; A61Q5/00(2006.01)i

According to International Patent Classification (IPC) or to both national classification and IPC

**B. FIELDS SEARCHED**

Minimum documentation searched (classification system followed by classification symbols)

IPC: C07K, A61K, A61Q

Documentation searched other than minimum documentation to the extent that such documents are included in the fields searched

Electronic data base consulted during the international search (name of data base and, where practicable, search terms used)

CNTXT, ENTXTC, ENTXT, OETXT, WOTXT, EPTXT, USTXT, ISI_Web of Science, CNKI, GenBank, EMBL, STN, 中国专利生物序列检索系统, China Patent Biological Sequence Search System: cyclic, peptide, cyclopeptide, pentapeptide, hexapeptide, heptapeptide, GHK, 三肽-1, Gly-His-Lys, 三胜肽, 蓝铜胜肽, cosmetic, hair, skin, 美容, 皮肤, 毛发, SEQ ID NO: 1

**C. DOCUMENTS CONSIDERED TO BE RELEVANT**

| Category* | Citation of document, with indication, where appropriate, of the relevant passages | Relevant to claim No. |
|---|---|---|
| A | NGYEN, H. et al. "Transition Metal Complexes of a Cyclic Pseudo Hexapeptide: Synthesis, Complex Formation and Catalytic Activities" *Journal Of Peptide Science,* Vol. 14, No. (9), 30 September 2008 (2008-09-30), 1010-1021<br>page 1014, figure 1, and pages 1015-1016, schemes 2 and 3 | 1-13 |
| A | GAHAN, L.R. et al. "Metal Complexes of Synthetic Cyclic Peptides" *Polyhedron,* Vol. vol. 153, 01 October 2018 (2018-10-01), 1-23<br>page 5, right-hand column, paragraph 2, and page 13, left-hand column, paragraph 1 | 1-13 |
| A | CZAPOR, H. et al. "The Cyclopeptides with the Multi-His Motif as Ligands for Copper(II)" *Journal of Inorganic Biochemistry,* Vol. 105, No. (2), 26 November 2010 (2010-11-26), 297-302<br>p. 297, abstract | 1-13 |
| A | US 2006183689 A1 (DENMEADE, S.R. et al.) 17 August 2006 (2006-08-17)<br>description, paragraphs 0017-0024, and SEQ ID NO: 52 | 1-13 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **20 December 2024** | **25 December 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/120959**

**C. DOCUMENTS CONSIDERED TO BE RELEVANT**

| Category* | Citation of document, with indication, where appropriate, of the relevant passages | Relevant to claim No. |
|---|---|---|
| A | CN 116120402 A (SHENZHEN WINKEY TECHNOLOGY CO., LTD.) 16 May 2023 (2023-05-16)<br>description, paragraphs 0009-0075 and 0105-0109 | 1-13 |
| A | 苏思佳等 (SU, Sijia et al.). "环肽合成策略及其在新药研发中的应用研究进展 (Research Progress of Cyclic Peptide Synthesis Strategy and its Application in New Drug Development)" *药学学报 (Acta Pharmaceutica Sinica),* Vol. 58, No. (3), 31 March 2023 (2023-03-31), 629-638<br>page 636, right-hand column, paragraph 1 | 1-13 |

Form PCT/ISA/210 (second sheet) (July 2022)

**INTERNATIONAL SEARCH REPORT**

International application No.
**PCT/CN2024/120959**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

INTERNATIONAL SEARCH REPORT

International application No.
PCT/CN2024/120959

**Box No. III Observations where unity of invention is lacking (Continuation of item 3 of first sheet)**

This International Searching Authority found multiple inventions in this international application, as follows:

Claim 1 sets forth a cyclic peptide compound represented by formula I or a stereoisomer thereof; claim 7 further defines that a cyclic peptide compound has a structure as shown in formula I-1; claim 9 defines that the structural formulae of cyclic peptide compounds are as shown in table A, and 729 different cyclic peptide compounds (SEQ ID NOs: 1-729) are listed in table A ; and claims 10-13 all directly or indirectly refer to claims 1-9. It can be seen therefrom that claims 1-13 comprise 729 inventions involving different cyclic peptide compounds. The structures of the 729 groups of cyclic peptide compounds are different, and the common technical feature thereof lies in comprising GHK. However, D1 (Transition Metal Complexes of a Cyclic Pseudo Hexapeptide: Synthesis, Complex Formation and Catalytic Activities, 30 September 2008) discloses a cyclic hexapeptide comprising a GHK structure (see figure 1, and schemes 2 and 3). It can be seen that the common technical feature of the 729 groups of cyclic peptide compounds is not a special technical feature. Therefore, the 729 inventions comprised in claims 1-13 and involving different cyclic peptide compounds do not have a special technical feature that defines a contribution which the inventions make over the prior art as defined in PCT Rule 13.2. Therefore, the present application lacks unity of invention (PCT Rule 13.1).

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-13 (in part)**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
**Information on patent family members**

International application No.
**PCT/CN2024/120959**

| Patent document cited in search report | | | Publication date (day/month/year) | Patent family member(s) | | | Publication date (day/month/year) |
|---|---|---|---|---|---|---|---|
| US | 2006183689 | A1 | 17 August 2006 | AU | 7627200 | A | 19 February 2001 |
| | | | | US | 2008247950 | A1 | 09 October 2008 |
| | | | | US | 2012309692 | A1 | 06 December 2012 |
| | | | | US | 8450280 | B2 | 28 May 2013 |
| | | | | EP | 1274723 | A2 | 15 January 2003 |
| | | | | EP | 1274723 | A4 | 01 October 2003 |
| | | | | WO | 0109165 | A2 | 08 February 2001 |
| | | | | WO | 0109165 | A3 | 07 March 2002 |
| | | | | WO | 0109165 | A8 | 20 June 2002 |
| CN | 116120402 | A | 16 May 2023 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311247312
- CN 202411310856


### Non-patent literature cited in the description


- Immunology-A Synthesis. Sinauer Associates, 1991